# EUROPEAN PATENT SPECIFICATION

(11) **EP 2 550 046 B1**
(45) Date of publication and mention of the grant of the patent: **24.04.2019**
(21) Application number: 11759906.8
(22) Date of filing: 14.03.2011
(51) Int. Cl.: A61M 5/315, A61B 17/00

(54) **DISPOSABLE SYRINGE APPLICATORS FOR MULTI-COMPONENT FORMULATIONS**
EINWEGSPRITZENAPPLIKATOREN FÜR FORMULIERUNGEN AUS MEHREREN BESTANDTEILEN
APPLICATEURS JETABLES À SERINGUE POUR DES FORMULATIONS À COMPOSANTS MULTIPLES

(30) Priority: 23.03.2010 US 316516 P; 14.01.2011 US 432832 P
(43) Date of publication of application: 30.01.2013
(73) Proprietor: Hyperbranch Medical Technology, Inc., Durham, NC 27713-4410 (US)
(72) Inventor: BUTLIN, Jared, D.G., Durham, NC 27713 (US); D'ALESSIO, Keith, R., Carey, NC 27511 (US)
(74) Representative: HGF Limited
(86) International application number: PCT/US2011/028301
(87) International publication number: WO 2011/119360

(56) References cited:
- EP-A1- 0 925 026
- EP-A1- 0 996 474
- WO-A1-97/20585
- US-A- 5 360 410
- US-A1- 2002 074 054
- US-A1- 2002 087 144
- US-A1- 2003 114 797
- US-A1- 2006 079 834
- US-A1- 2008 161 757
- US-A1- 2009 062 741
- US-A1- 2010 010 473
- US-B1- 6 565 539

## Description

### BACKGROUND

A number of medically useful compositions comprise two or more ingredients that for optimal results should not be mixed together until shortly prior to use. In some instances, at least one of the ingredients is a solid, often a powder, while at least one of the other ingredients is a liquid in which the solid ingredient is to be dissolved. Therefore, it is desirable to have an applicator that can easily deliver multiple-component formulations for use in the body, which applicators are capable of keeping the individual components separated during storage and mixing them prior to application.

Use of a dual-ingredient composition can be accomplished with a conventional syringe by first loading one ingredient into the syringe, then adding the second ingredient, shaking the syringe or otherwise agitating the contents to achieve mixing, and subsequently dispensing the resulting mixture in the usual manner. This procedure, however, presents substantial shortcomings, including contamination and loss of sterility. For example, using a conventional syringe of the kind that is filled through a fill needle connected to the outlet orifice of the syringe, it is necessary to replace the needle after the first ingredient has been drawn into the syringe in order to avoid contamination of the supply of the second ingredient. Even then it may be difficult to complete the procedure without rendering the outlet portion of the syringe non-sterile, e.g., by extended contact with air. Another technique that may be employed utilizes a syringe of generally conventional construction in which one ingredient has initially been loaded into the syringe, usually followed by a sterilization procedure. Again, however, it is often rather difficult to load the syringe with the second ingredient without undermining the sterile characteristics of the syringe. Moreover, in both of these procedures the user's manipulative steps are complex enough that some difficulty may be experienced.

One approach to this problem is described in US Patent No. 5,080,649. Therein is described a hypodermic syringe which has an elongated tubular body having a front end adapted to carry a needle, a rear end, and a bypass between the ends, wherein a front partition piston defines with the front end a front compartment adapted to hold a substance and a rear piston defines with the front piston a rear compartment adapted to hold a fluid miscible with the front-compartment substance; and the front piston is displaceable into a middle position in the bypass for fluid communication between the compartments. See, also, US Patent Application Publication No. 2007/0276322, which relates to syringe devices and methods of mixing and administering medication.

Some medical sealants are examples of medically useful compositions which comprise two or more ingredients that are not mixed together until shortly prior to use. Medical sealants and adhesives play an important role in helping patients recover from surgery or trauma. In particular, sealants and adhesives are useful in treating patients suffering from a variety of internal or topical conditions, including lacerations, tears, wounds, ulcers, anastomoses, and surgical procedures. Sealants or adhesives can generally be used in any indication or application for which a suture or staple is presently used, and the sealant or adhesive often provides a better outcome than a suture or staple. Sealants or adhesives can also be applied more quickly to the injury site and often provide a better seal over the wound, and ultimately improved healing, in comparison to a conventional suture or staple.

There are at least two medical sealant/adhesive products, CoSeal and DuraSeal, currently on the market which are based on hydrogel formulations. Both products comprise multiple components housed in separate containers. CoSeal Surgical Sealant (CoSeal) is composed of two synthetic polyethylene glycols (PEGs), a dilute hydrogen chloride solution and a sodium phosphate/sodium carbonate solution. The DuraSeal Dural Sealant System consists of components for preparation of a synthetic, absorbable sealant and an applicator for delivery of the sealant to the target site the sealant is composed of two solutions, a polyethylene glycol (PEG) ester solution and a trilysine amine solution. However, the products have shortcomings because the devices need to be assembled at the time of use and they utilize static mixing systems that allow the hydrogel formulation to gel within the mixing nozzle (dispensing tip), precluding a start-and-stop application technique.

Fibrin glues are also sold in packaging and applicator systems that are similar to those used for CoSeal and DuraSeal. One example is Baxter's Tisseel. Tisseel VH [Fibrin Sealant] consists of a two-component fibrin biomatrix that offers highly concentrated human fibrinogen to seal tissue and stop diffuse bleeding.

Baxter also offers different types of applicators, for example, Duploject; Easyspray; and DuploSpray MIS. Duploject is a reconstitution device that offers needle free easy preparation. Easyspray is a disposable set consisting of a dual-lumen connector hose, a sterile filter, two spray heads and a clip to be attached to the Duploject plunger for gas activation. DuploSpray MIS applicator is a disposable spray applicator consisting of a stainless steel shaft, dual lumen spray tubing, sterile filter and two replaceable spray tips.

In addition, Micromedics, Inc. a medical device manufacturer in St. Paul, MN, manufactures an endoscopic spray system for biomaterials called the FibriJet. FibriJet incorporates a gas-assisted spray system. Spraying of fibrin glues is also discussed in the patent literature; see: US Patent Nos. 5,474,540; 4,874,368; and 5,368,563. See also, US Patent No. 4,735,616, which describes a twin bypass syringe for the delivery of fibrin glue products.

US 2002/0074054 A1 relates to a device for storing a liquid medicinal substance, particularly a component of a multi-component tissue adhesive.

EP 0925026 A1 relates to an applicator device for applying a multi component fluid, especially a multi component tissue glue.

US 6565539 B1 relates to a device for applying a flowable medium, in particular a single- or multiple-component tissue adhesive.

### SUMMARY

According to the present invention, there is provided an applicator as defined in claim 1.

According to an aspect of the present invention, there is provided a kit as defined in claim 8.

Certain aspects of the invention relate to an applicator which can house multiple components of a formulation in separate receptacles, and which can be utilized to combine the components at the time of use to form the formulation.

In certain embodiments, a device of the invention can be used for, but is not limited to, applying hydrogel formulations to dura mater (dura), abdominal tissue in hernia repair, tissues near the spine, lung tissue, intestinal tissue, and any of the internal tissues. In certain embodiments, a device of the invention can be configured to apply a spray or a stream of liquid formulation onto a surface to be treated. In certain embodiments, a device of the invention can be configured to deliver the formulation through a trocar in a scope (e.g., an endoscope or laparoscope).

One aspect of the invention relates to an applicator that can be used to house separately liquids, viscous liquids and solids (*e.g*., components of a polymerizable hydrogel), which is further designed to facilitate the combination of the liquids, viscous liquids and/or solids inside the applicator, and is also designed to facilitate the application of the resulting mixture to a surface. In certain embodiments, such an applicator may be used for delivering a composition to a tissue. For example, such an applicator may be used for delivering a formulation to the dura mater or a cornea. In addition, the applicators may be useful for a variety of other applications, including, for example, preparation and application of a vascular sealant or arterial access closures.

### BRIEF DESCRIPTION OF THE FIGURES

Certain embodiments of the invention are described below with reference to the following accompanying figures.
**Figure 1** depicts a photograph of an applicator.
**Figure 2** depicts a photograph of several exemplary applicator tips.
**Figure 3** depicts a photograph of an applicator not forming part of the present invention.
**Figure 4** depicts photographs showing selected steps in opening and assembling an applicator.
**Figure 5** depicts photographs showing selected steps for using an applicator.
**Figure 6** depicts [**A**] an applicator and vial; and [**B**] the tip of the applicator being inserted through the septum on the vial.
**Figure 7** depicts photographs showing selected steps in opening and assembling an applicator.
**Figure 8** depicts an exploded view of a syringe barrel with a stepped bore, a dissolution aid and a retainer ring.
**Figure 9** depicts a syringe barrel with a stepped bore, with a dissolution aid and a retainer ring at its distal end.
**Figure 10** depicts two views of a retainer ring.
**Figure 11** depicts a syringe barrel, with a dissolution aid and sleeve insert (which when ultrasonically welded in place towards the distal end of the barrel creates a stepped bore).
**Figure 12** depicts an exploded view of a syringe barrel, with a dissolution aid and sleeve insert (which when ultrasonically welded in place towards the distal end of the barrel creates a stepped bore).
**Figure 13** depicts a syringe barrel, with a dissolution aid and sleeve insert (which when ultrasonically welded in place towards the distal end of the barrel creates a stepped bore).
**Figure 14** depicts one embodiment of the sleeve insert.
**Figure 15** depicts a prototype applicator of the invention, including two liquid filled syringes, an internal micro air pump and internal battery pack, two vial adapters containing valves which have a spike for use in accepting rubber-septum-sealed glass vials, and two caps sealing and protecting the vial adapter spikes.
**Figure 16** depicts the prototype applicator of Figure 15 with the PEI- or PEI solution-containing vial installed onto the PEI-side vial adapter spike, and the activated-PEG-containing vial installed onto the PEG-side vial adapter spike.
**Figure 17** depicts the prototype applicator of Figures 15 and 16 in the form (e.g., with both reagents reconstituted and their respective vials removed) in which it is ready for use in the sterile field.
**Figure 18** depicts schematic drawings of top and side views of a prototype applicator of the invention (with cylindrical housings covering the vial adapters). *See* Figure 15.
**Figure 19** depicts schematic drawings of top and cross-sectional side views of a prototype applicator of the invention (with cylindrical housings covering the vial adapters). *See* Figure 15.

### DETAILED DESCRIPTION

There is a need to develop improved medical dispensers that facilitate the complete mixing of solids and/or liquids inside the dispenser while maintaining the sterility of the mixture. In addition, there is need for medical dispensers that allow two or more components which are to be mixed to be kept separate until just prior to use. Further, it would be advantageous if the dispensers could also act as applicators, thereby facilitating the application of the mixture. The present invention addresses these needs and others.

One aspect of the invention relates to an applicator system that may be used to house multiple components (e.g., components of a polymerizable hydrogel, such as solids and liquids), facilitating the mixing of the components inside the applicator, and further facilitating the application of the resulting mixture. Another aspect of the invention relates to an applicator system that may be used to house components of a polymerizable hydrogel, facilitating their mixing and further facilitating the application of the mixture.

While the invention will often be described herein as facilitating the formation and effective delivery of a polymerizable hydrogel formulation to a patient, this characterization is not intended in any way to limit the scope of the invention to such an application. Rather, the applicators of the invention, may be used in any application requiring mixing two, three or more components (e.g., solids, liquids) prior to use. It is understood that these applicators may be useful for a variety of applications including, for example, treating/sealing/adhering dura mater, cardiovascular tissue, ducts, bladders, lung tissue, liver, other parenchymal organs, as well as adhering soft tissue mesh to the body.

In certain embodiments, the applicators of the invention can be used to prepare and apply a hydrogel formulation. In certain embodiments, the hydrogel formulation is delivered in liquid form and quickly polymerizes into a hydrogel. In certain embodiments, the hydrogel formulation comprises a cross-linking agent (such as PEI); a polymerizable material (such as activated PEG); and a buffer solution or solutions. In certain embodiments, drug(s) and/or medicament(s) could be added as a formulation part or formulation part component(s). In certain embodiments, the various components are kept separate and remain stable during their intended shelf life.

One aspect of the invention relates to applicators. In certain embodiments, the applicator is made up of a syringe barrel containing an aqueous solution which is connected to a valve assembly. One such valve assembly is described in U.S. Patent No. 6,238,372. In certain embodiments, the aqueous solution is a buffer. In certain embodiments, the solution contains a cross-linking agent. In certain embodiments, the valve assembly is configured so that one path of the valve assembly connects the syringe barrel to a mixing chamber which contains a solid. In certain embodiments, the mixing chamber is a serum vial, and the valve assembly includes a spiked vial adapter. In such applicators the valve allows fluid from the syringe barrel to pass through the spiked vial adapter into the mixing chamber. In certain embodiments, the fluid can then be drawn back into the syringe barrel and reintroduced into the mixing chamber several times to allow for homogeneous mixing of the liquid and the solid. When the mixing is complete the solution can be drawn into the syringe, the valve can be switched to allow for delivery of the mixture, and the mixture can be delivered to a surface. In some instances, removing the adapter simultaneously opens the valve for delivery of the mixture to the surface.

An applicator of the present invention comprises a gas pump, wherein said gas pump assists with fluid movement within the applicator or from the dispensing tip or both. The gas pump assists with fluid movement from the dispensing tip. In certain embodiments, the dispensing tip is a gas-assisted spray tip. In certain embodiments, the gas pump is an air pump. In certain embodiments, the gas pump is battery powered, wherein said battery or batteries are built-in or removable. In certain embodiments, said battery or batteries are removable. The gas pump is a built-in component of the applicator.

In certain embodiments, an applicator of the present invention further comprises a regulator and a compressed gas cylinder, wherein said compressed gas is released to assist with fluid movement within the applicator or from the dispensing tip or both. In certain embodiments, the compressed gas assists with fluid movement from the dispensing tip. In certain embodiments, the compressed gas assists with fluid movement from the dispensing tip, wherein the dispensing tip is a gas-assisted spray tip. In certain embodiments, the compressed gas is air, nitrogen, a hydrofluoroalkane, or nitrous oxide. In certain embodiments, the compressed gas is air or nitrogen. In certain embodiments, the compressed gas is air. In certain embodiments, the compressed gas cylinder is a built-in component of the applicator. In certain embodiments, the compressed gas cylinder is removable.

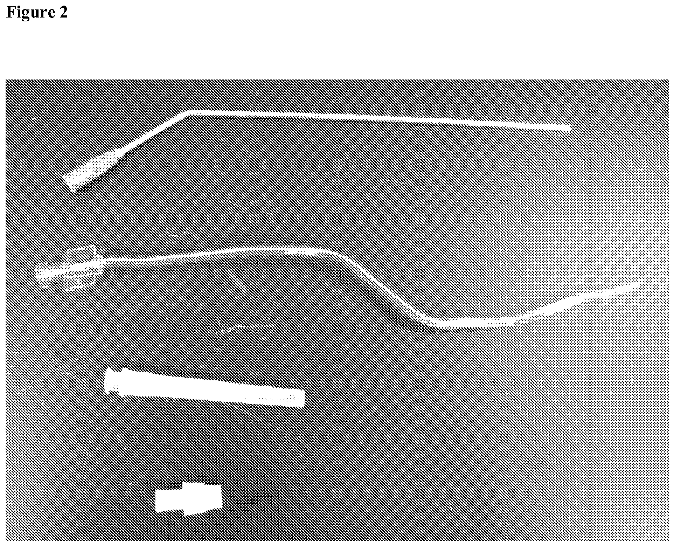

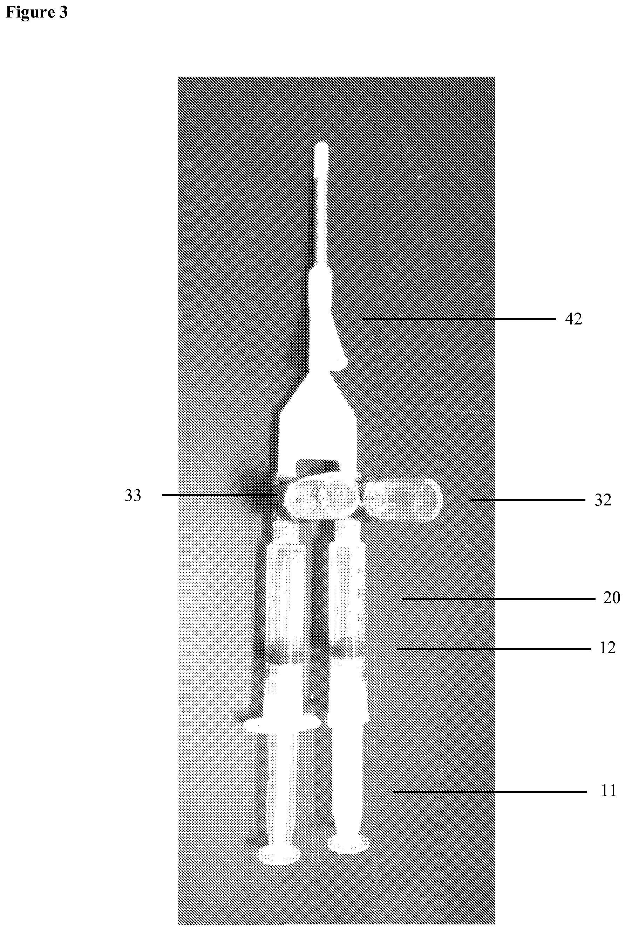

In certain embodiments, the applicator may include a variety of dispensing tips (such as those shown in Figure 2). In some instances the tips may be but are not limited to: a brush tip, spray tip, gas-assisted spray tip, cannula, needle, flexible tube, bent cannula, shape conforming tubing, or any combination thereof. In certain embodiments, the tip allows for multiple devices to be connected and delivered, as shown in Figure 3.

In certain embodiments, a filter is present in the syringe body to reduce the introduction of gas bubbles. In certain embodiments, the filter is coated with a cross-linking agent and the filter thereby acts as a dissolution aid. In certain embodiments, the filter can be used to divide the syringe barrel into two compartments, and serve as a hydrophobic barrier separating the two compartments. In certain embodiments, the syringe barrel has a retention ring molded into the barrel to immobilize the filter.

In some instances the syringe barrel, starting from the distal end moving toward the proximal end contains a bottom chamber and a top chamber. The bottom chamber is defined by the distal end of a syringe barrel and a hydrophobic barrier. The bottom chamber holds a cross-linking agent. In certain embodiments, the cross-linking agent is impregnated on a dissolution aid, such as a porous filter. In certain embodiments, the cross-linking agent is polyethyleneimine. The top chamber defined by a plunger or pushrod at the proximal end of the syringe barrel and a hydrophobic barrier. The top chamber holds a buffer. The barrier separates the cross-linking agent and the buffer solution when the device is stored. In certain embodiments, the hydrophobic barrier can allow fluid to pass when actuated by the user. In certain embodiments, the barrier is a hydrophobic filter of varying density. In other embodiments, the barrier is a valve.

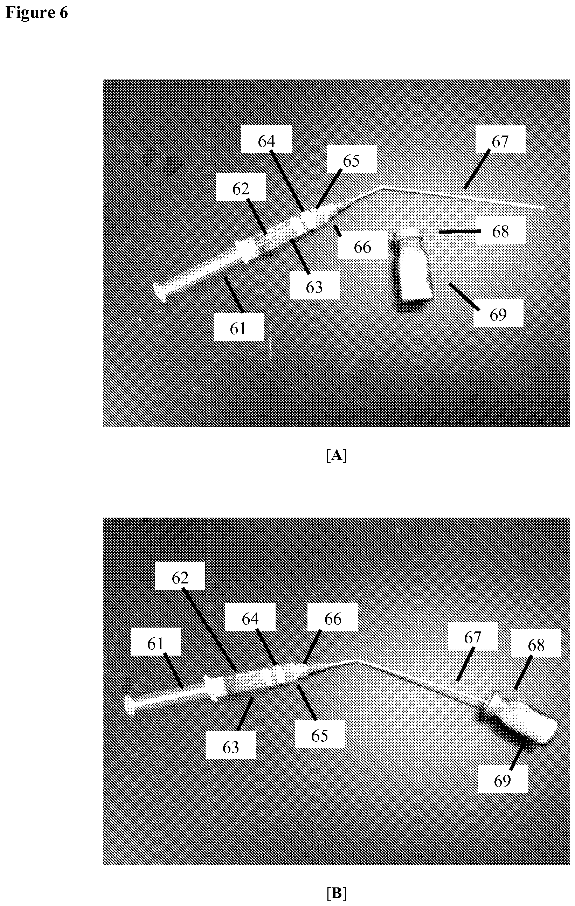

Applicators such as the one shown in Figure 6 consist of a liquid filled syringe, a powder containing vial, and an appropriate tip (such as a long cannula). In certain embodiments, the vial incorporates a slit septum which allows the blunt cannula to easily penetrate and come into fluid communication with the contents of the vial. In certain embodiments, the pressure exerted by collar on the septum keeps the slit closed.

Another aspect of the invention relates to applicators with improved ways of holding a dissolution aid in place. A problem with some of the traditional ways of holding a dissolution aid in place is that one would need a protuberance on the inner bore surface of the syringe (i.e., a protuberance which is molded into the sidewall of the syringe). In certain embodiments, this can be very difficult to manufacture via a normal injection molding process, as the protuberance is an undercut to the surface of the core pin which is defining the inner surface of the bore of the syringe. Such undercuts are difficult to manufacture as they capture the core pin. However, in some syringes a small rounded undercut is sometimes placed close to the proximal end (in order to provide some resistance to complete backing out of the syringe plunger) and although not ideal from a manufacturing standpoint, it can be accomplished by removing the core pin while the part is still hot and somewhat compliant. Such a plunger retention undercut is also possible because it is located at the proximal end of the syringe where it's easier to push against the flange, which is in close proximity to the plunger retention ring. In the case of a dissolution aid retention protuberance or ring, since it is most desirable for the dissolution aid to be in the most distal end of the syringe body, the ring must be far from the flange, thus decreasing the possibility of buckling of the syringe body wall during core pin extraction.

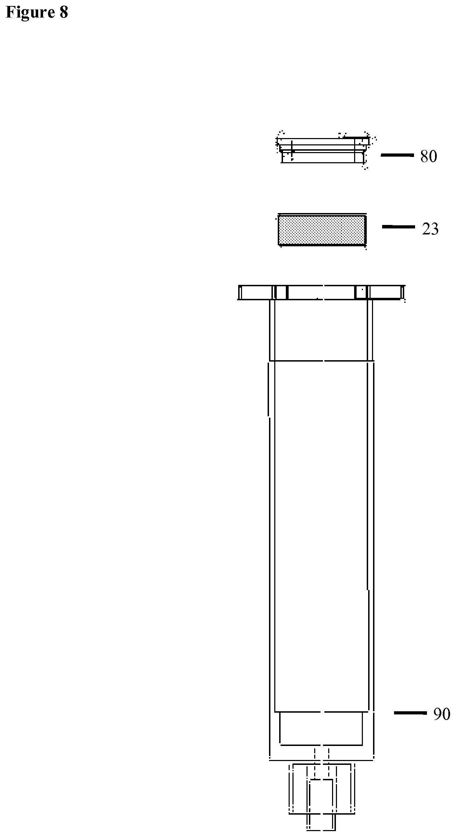

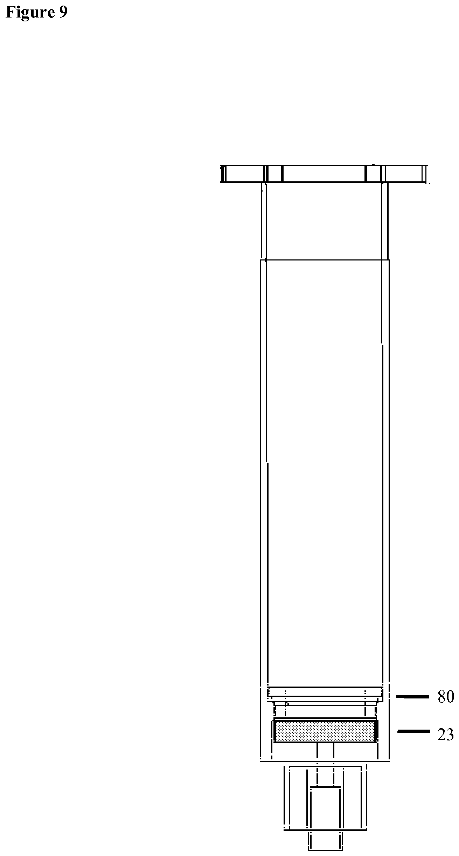

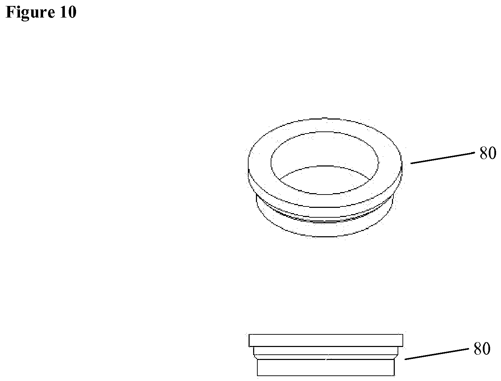

Figures 8 and 9 show a two part custom syringe barrel with a stepped bore (90), dissolution aid (23) and retainer (80) which do not suffer from having to be manufactured with undercuts in the core pin. Upon close examination of the distal bore of the syringe barrel, one will notice that the bore has a slightly smaller inner diameter in the most distal interior aspect than the rest of the bore. In certain embodiments, a retainer (80) is designed to be ultrasonically welded in place behind the dissolution aid, a filter or the like, which is thereby held in place at the distal end of the barrel by the retainer. Figure 8 is an exploded view of one such stepped bore subassembly. Figure 9 shows the same subassembly, with all of the pieces in place, after ultrasonic welding of the retainer in place over the step of the stepped bore. In certain embodiments the retainer has a central through hole allowing liquid to easily flow through the dissolution aid, filter or the like during use (see Figure 10).

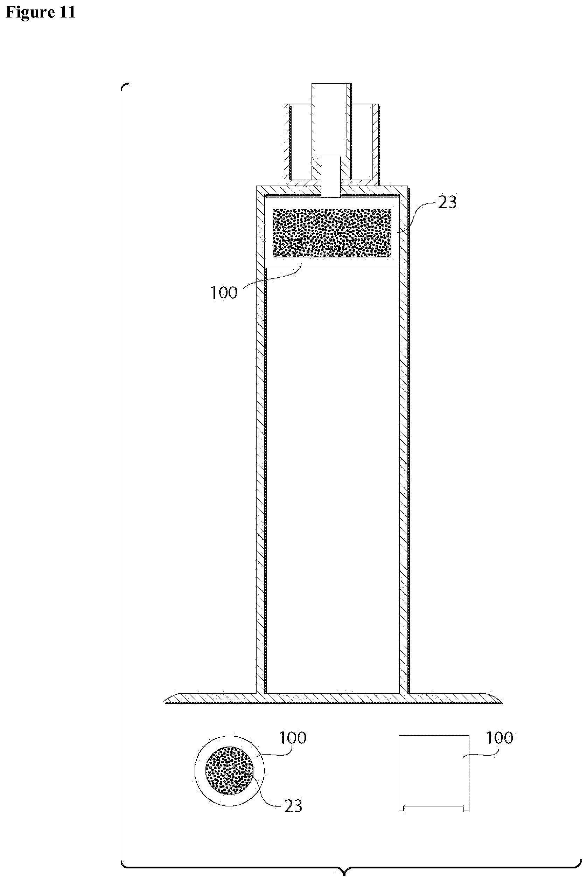

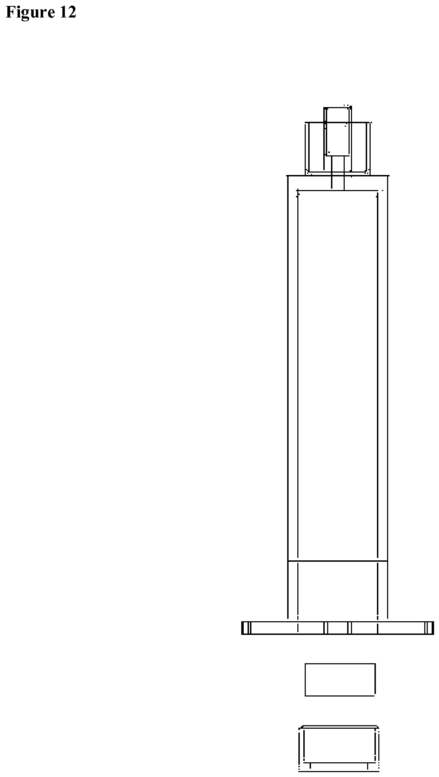

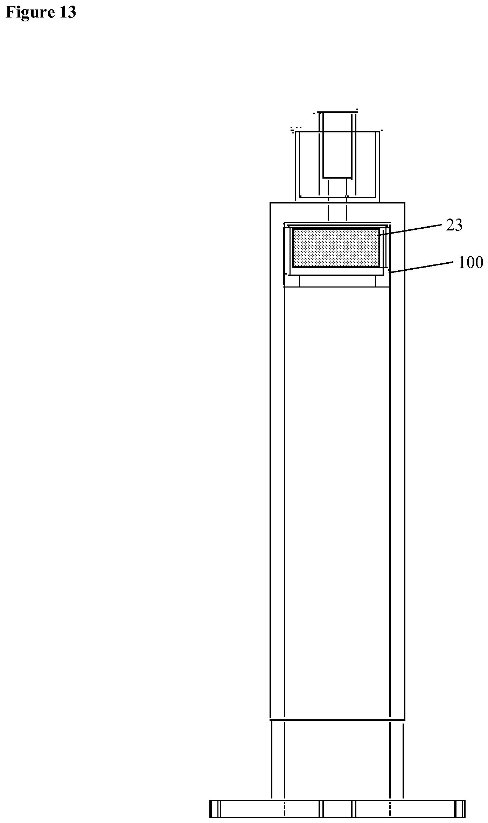

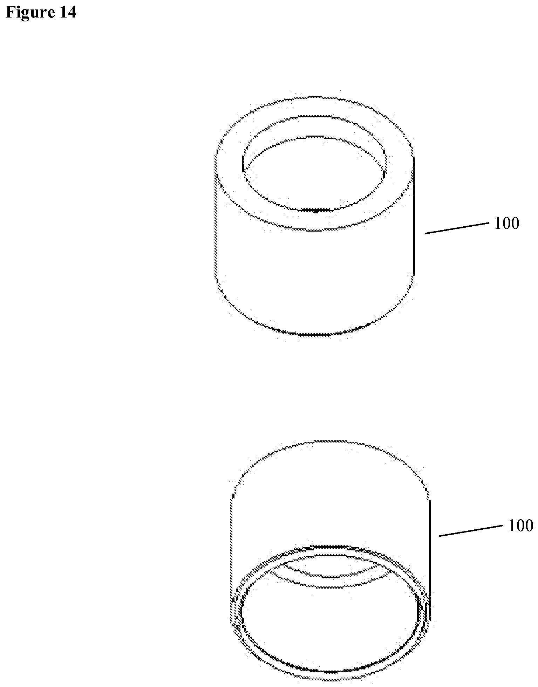

In certain embodiments, instead of using a custom molded stepped bore syringe as described above, a sleeve insert (100) can be placed within a standard syringe, such that it creates a stepped bore, such that the step that is formed can aid in retaining a dissolution aid (23) or filter (see Figures 11-14). In certain embodiments, the sleeve insert can be ultrasonically welded to the interior aspect of the syringe barrel. In certain embodiments, the sleeve insert further comprises energy directors (101) at its distal end; these energy directors are protrusions from the sleeve insert which assist in the ultrasonic welding of the sleeve to the interior aspect of the syringe barrel.

In certain embodiments, the applicators described herein are able to produce frothing, thereby decreasing the density of the material (e.g., a hydrogel) dispensed from the applicators relative to its original density. As used herein, "frothing" refers to the incorporation of gas, such as air, into a material, thereby decreasing its density. In certain embodiments, the density of the frothed material can be reduced by about 25%, about 50% or about 75%, compared to its original density. In certain embodiments, the density of the frothed material can have a bulk density of less than water (i.e., <1 g/cc). In certain embodiments, the bulk density of the frothed material is between about 0.1 g/cc and about 1 g/cc, 0.2 g cc and about 0.9 g/cc, 0.3 g/cc and 0.8 g/cc, 0.4 g/cc and about 0.7 g/cc or about 0.45 g/cc and about 0.68 g/cc. In certain embodiments, the material is a hydrogel.

In some instances, frothing may be desirable. For example, frothing can reduce the amount of a material a doctor may use, and implant in the body, resulting in a lesser mass effect. Reduced residence time may also is achieved by using less material. In addition, the frothing can also be used as a way to increase hydrolytic cleavage and further reduce the residence time of a material in the body by increasing the surface area in contact with bodily fluids responsible for the cleavage. Further, frothing can reduce/prevent ingress of the material applied into a wound site and, in the case of dural repair, into the CSF. It is believed the reduction of ingress is due to the decreased density of the material and the resulting tendency of the material to float, as well as on account of an increase in the viscosity of the material applied, which reduces its miscibility with bodily fluids.

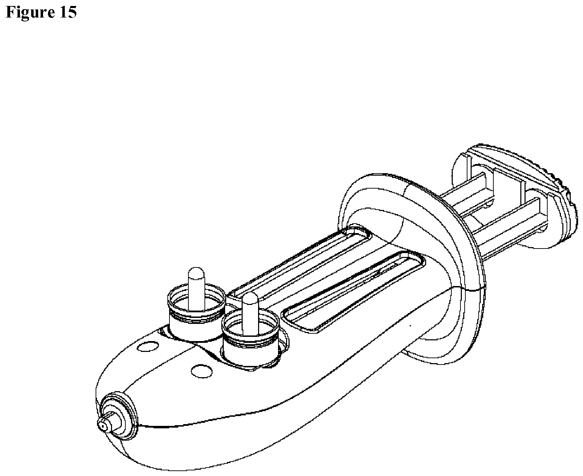

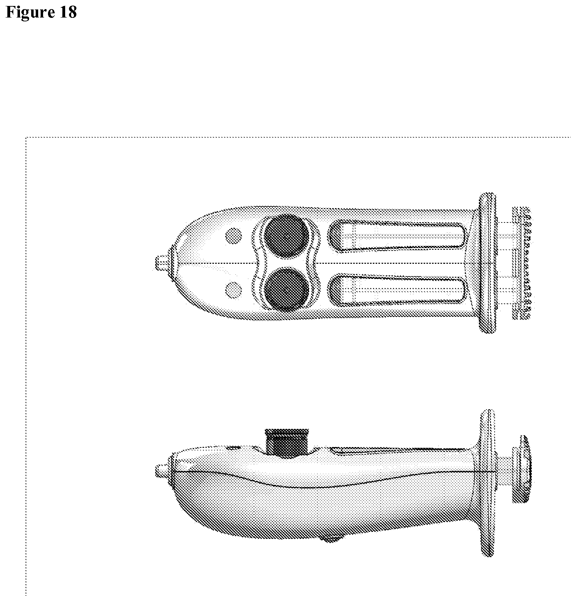

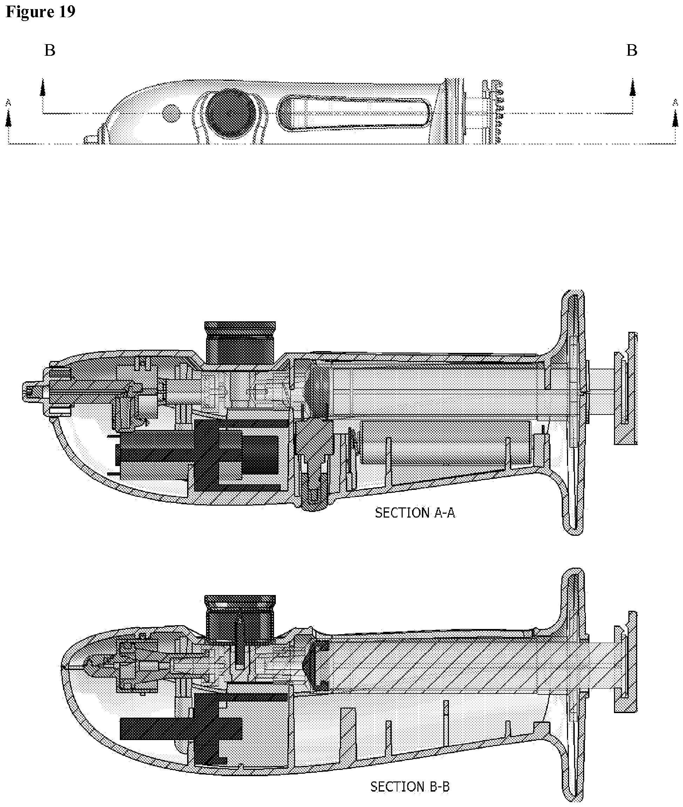

In certain embodiments, such as those depicted in Figures 15-19, a number of the components of the applicator are contained within a housing. In certain embodiments, said housing comprises the dispensing tip at a first proximate end, and an opening or openings at a first distal end through which the syringe barrel or barrels may pass. In certain embodiments, said housing is fabricated from acrylonitrile butadiene styrene, polypropylene, or isoprene.

### Polyalkyleneimine Hydrogels

In one aspect of the present disclosure relates to applicators for polyalkyleneimine hydrogels, and methods for using such applicators. Polyalkyleneimine hydrogels can be prepared by reacting a polyalkyleneimine (PAI) with a cross-linking agent, such as an activated polyethylene glycol. Polyalkyleneimine hydrogels are amendable to a variety of clinical treatments, such as incisions created during general surgery or wounds/incisions in the dura created during neurosurgery. Polyalkyleneimine hydrogels offer the advantage that the secondary and tertiary amino groups of the gel can be converted to secondary and tertiary ammonium cations which may encourage cell attachment and cell ingrowth. In certain instances, the secondary and tertiary amines of the polyethyleneimine (PEI) can be converted to ammonium cations by placing the PEI in an aqueous solution.

Polyalkyleneimine (PAI) hydrogels are known to have superior adhesion properties. Their superior tissue-adhesion properties may be due to two factors. First, the cationic properties of PEI promote interaction with, and possibly penetration within, an anionic tissue substrate. See Rep. Prog. Phys. 1998, 61, 1325-1365. Cationic interactions could occur through the secondary and tertiary ammonium cations of the PEI backbone or through primary amino groups that did not react with the cross-linking reagent. Second, PEI contains a large number of functional groups per molecule, thus promoting an increased number of cross-linkable sites within the polymer network. The increased number of cross-linkable sites within the polymer network affords dense, interpenetrating networks between the hydrogel and the tissue surface. The number of free amino groups in the hydrogel can be controlled by varying the ratio of PEI to activated PEG. The ability to control the number of free amino groups is significant because greater cell ingrowth was observed in tissue ingrowth experiments using hydrogels that contained a larger percentage of PEI.

In addition to increased adhesion, it has been found that as the molecular weight of the PEI increases from about 1,300 to about 2,000 g/mol the swelling of the resulting hydrogel decreases in certain instances. Thus, the molecular weight of the PEI may be adjusted in order to tune the swelling-effects of the resultant hydrogel.

A large variety of PAI derivatives are amenable to the present disclosure. For example, the amino groups of the PAI may be functionalized with a fatty acid, lower alkyl, an alkenyl, or alkynyl group. In addition, the amino groups or a portion of the amino groups may be functionalized to contain active agents, pharmaceutical agents, preservatives, radio isotopic ions, magnetically detectable ions, antibodies, medical contrast agents, colorants, dyes, or other visualization agents. In certain instances, about 1% to about 70% of the primary amines of the PEI are functionalized. The PAI derivatives may contain hydrolytically and/or enzymatically degradable linkages capable of releasing the functional derivatives, active agents, pharmaceutical agents, preservatives, radio isotopic ions, magnetically detectable ions, antibodies, colorants, dyes, or other visualization agents. Alternatively, a different nucleophile can be added to the PEI, such as a cysteine, isocysteine, thiol, or other such nucleophilic group. For example, a PEI can be modified such that all the primary amines are modified with a cysteine thus affording a PEI derivative which can form cross-linked gel/networks using the amine, thiol, or both the amine and thiol. In certain instances, an ureido, urea, acetoacetoxy, RGD peptide, EDTA, or carbohydrate group may be bonded to one or more of the amino groups of the PEI. Representative carbohydrates include erythrose, threose, ribose, arabinose, xylose, lyxose, allose, altrose, glucose, mannose, gulose, idose, galactose, talose, sucrose, lactose, and the like. It is possible that the ureido group and urea group will impart adhesion partially via a cation/anion interaction. The acetoacetoxy group may adhere to tissue by making a metal complex on the surface of the tissue.

In certain instances, the PEI is functionalized so that both primary amino (-NH2) groups and thiol (-SH) groups could react with electrophilic groups or a combination of them, such as an acrylate, succinimidyl ester, maleimide, ester, or aldehyde. The electrophilic groups can be attached to poly(alkyleneoxide) (e.g., PEG, PPG or PEG-PPG) polymers. Two or more electrophilic groups are required. Of course, the degree of PEI functionalization may be varied in order to obtain the desired physical properties of the resultant gel. In certain instances, only about 1% of the primary amino groups of the PEI are functionalized. In other instances, about 5% to about 25% of the primary amino groups of the PEI are functionalized. In other instances, about 25% to about 50% of the primary amino groups of the PEI are functionalized. In other instances, about 99% of the primary amino groups of the PEI are functionalized. In certain instances, one or more of the amino groups are reacted with an epoxide or acylating agent. In certain instances, one or more of the amino groups are reacted with an isocyanate.

The molecular weight of the PEI may be adjusted to tune the physical properties of the gel formed by addition of the cross-linking agent. In certain instances, the PEI has a weight average molecular weight of about 400 g/mol to about 2,000,000 g/mol. In certain instances, the PEI has a weight average molecular weight of about 400 g/mol to about 1,000,000 g/mol. In certain instances, the PEI has a weight average molecular weight of about 400 g/mol to about 500,000 g/mol. In certain instances, the PEI has a weight average molecular weight of about 400 g/mol to about 100,000 g/mol. In certain instances, the PEI has a weight average molecular weight of about 400 g/mol to about 50,000 g/mol. In certain instances, the PEI has a weight average molecular weight of about 400 g/mol to about 10,000 g/mol. In certain instances, the PEI has a weight average molecular weight of about 400 g/mol to about 5,000 g/mol. In certain instances, the PEI has a weight average molecular weight of about 400 g/mol to about 2,000 g/mol.

In certain instances, the polyalkyleneimine has a weight average molecular weight of about 600 to about 10,000 Daltons, the polyalkylene glycol has a weight average molecular weight of about 500 to about 20,000 Daltons, and the molar ratio of the polyalkyleneimine to the polyalkylene glycol is within a molar range of about 0.025:1 to about 0.4:1. In certain instances, the hydrogel reaches equilibrium swelling in about 5 to about 30 hours. In certain instances, the hydrogel reaches equilibrium swelling in about 18 hours.

In certain instances, the aforementioned polyalkyleneimine / polyalkylene glycol hydrogels may be used or modified to non-covalently carry or contain active agents, pharmaceutical agents, preservatives, radioisotopic ions, magnetically detectable ions, antibodies, medical contrast agents, colorants, dyes, or other visualization agents.

Many prior sealant systems are not optimal because the sealant system may degrade before appreciable healing or tissue ingrowth occurs. For example, tissue ingrowth often begins within one week after application of the sealant, and complete tissue ingrowth may occur within 28 days after application of the sealant in porous systems. However, many prior sealant systems contain degradable linkages which can cause the hydrogels to degrade before appreciable tissue ingrowth occurs. While use of these materials alone is not advantageous, these materials may be used as masking materials. Accordingly, in certain instances, when polyalkyleneimine hydrogel are used as covering materials the covering can maintain its mechanical strength for at least about 7 days. In certain instances, the polyalkyleneimine hydrogel sealants of the disclosure maintain mechanical strength for at least about 20 days. This rate of degradation allows the masking material to degrade, while keeping the covering material in place.

Since charged species encourage tissue growth, polyalkyleneimines as masking material are advantageous because they allow for incorporation of a large number of charged species. The charged species are created by converting unreacted primary amines, and internal secondary and tertiary amines into ammonium cations under physiological conditions. Table 1 below illustrates the number of primary, secondary and tertiary amines contained in various cross-linkers based on a polymer system having eighteen primary amines. As illustrated in Table 1, the trilysine cross-linker contains only primary amines and a pendant carboxylate while a PPI(DAB)-G1 dendrimer adds 9 units of potential cationic charge with the addition of 9 tertiary amines. The PEI₈₀₀ adds 14 units of potentially charged species (i.e., 155% more charge) compared to the PPI(DAB)-G1 dendrimer, while the PEI₂₀₀₀ adds 26% more potentially charged species than PEI₈₀₀. Finally, PEI₂₅₀₀₀ adds 24% more potentially charged species than PEI₂₀₀₀, owing to the increased number of secondary and tertiary amines. Since the number of secondary and tertiary amino groups increases with increasing molecular weight of the polyalkyleneimine, the polyalkyleneimine hydrogels of the disclosure can be tuned by incorporating cross-linkers with varying molecular weights, and hence charge density, in order to affect the tissue ingrowth and degradation properties of the hydrogel.

**Table 1**

| Cross-linker | 1° amines | 2° amines | 3° amines |
|---|---|---|---|
| PEI₂₅₀₀₀ | 18 | 22 | 14 |
| PEI₂₀₀₀ | 18 | 17 | 12 |
| PEI₈₀₀ | 18 | 14 | 9 |
| PPI(DAB)-G1 | 18 | 0 | 9 |
| Trilysine | 4 | 0 | 0 |

Again, when used as masking material, polyalkyleneimine hydrogel sealants offer an advantage over prior sealant systems because polyalkyleneimines, especially derivatized polyalkyleneimines, should have antimicrobial and antiviral activity. Recent reports indicate that both polyalkyleneimines and derivatives thereof have antimicrobial properties, while lacking activity against mammalian cells. See Biotechnol. Bioeng. 2005, 90, 715-722; Biotechnol. Bioeng. 2003, 83, 168-172; Biotechnology Letters 2003, 25, 1661-1665; Biotechnol. Prog. 2002, 18, 1082-1086; Chem. Commun. 1999, 1585-1586; and Proc. Nat. Acad. Sci. USA 2006, 103, 17667-17671. Thus, hydrogels prepared from polyalkyleneimines may help fight, inhibit, prevent or even eliminate the chance for infection when applied to the tissue of a patient. Since the presence of cationic groups, especially quaternary amines, may influence the antimicrobial properties of the hydrogel, the PAI, in certain instances, may be derivatized with one or more quaternary amines. In certain instances, the PAI may be derivatized with four or more quaternary amines. In certain instances, the PAI may be derivatized with ten or more quaternary amines. Since the presence of cationic groups and hydrophobic side chains, when combined, tend to confer better antimicrobial properties, the PAI, in certain instances, may be derivatized with one or more quaternary amines and one or more fatty acid, lower alkyl, alkenyl, or alkynyl groups.

Polyalkyleneimine hydrogels offer the additional advantage that the amino groups of the polyalkyleneimine can act as a buffering agent. The ability to control the pH during preparation of the hydrogel is important because certain pHs are optimal for cross-linking of the components. In particular, the pH of a mixture of cross-linking components can affect the rate at which the cross-linking reaction takes places. In some instances, the desired pH can be achieved by adding a buffering agent, such as phosphates, carbonates, borates, and the like, to the solution containing the cross-linking components. However, when using poly alkyleneimines as a cross-linkable component, the primary, secondary, and tertiary amines act as buffering agents to provide some buffering capacity throughout a wide range of pHs. See Bioorganic Chemistry 1994, 22, 318-327. Moreover, as the cross-linkable component reacts, some of the amines are removed from solution, thereby reducing the pH. Since quick set-times can require higher pHs, it is advantageous to use a cross-linkable component which influences the pH so that the pH will lower to more physiological levels soon after mixing. This buffering feature of polyalkyleneimines eliminates the need for a strong buffer to achieve the high pH-levels sometimes used in preparing a hydrogel. Notably, addition of strong buffers may not be desirable because such buffers may remain in the sealant and cause the patient's tissue to become irritated.

As mentioned above, in certain embodiments the applicators of the invention may be configured to react polyalkyleneimines, or other amine-containing polymers, with cross-linking agents, to form hydrogels. A large number of cross-linking agents are amenable to the invention. In certain instances, the cross-linking agent is an activated polyethylene glycol. The activating group is preferably an electrophilic group. For example, in certain instances, the polyethylene glycol contains a N-hydroxysuccinimide group at each end of the polymer. In certain instances, the succinimide is functionalized with a sulfonic acid moiety. In certain instances, the polyethylene glycol contains an aldehyde at each end of the polyethylene glycol. In certain instances the polyethylene glycol is a star, dendritic, or branched polymer with three or more activating groups.

In certain instances, the polyethylene glycol cross-linking agent contains two or more different electrophiles. The different electrophiles may have similar or dissimilar reactivities. The different electrophiles provide linkages having similar or dissimilar degradation rates. The selection of electrophiles allows for control over the cross-linking reactions to form the hydrogels, the adhesive properties, and the degradation rate of the formed hydrogel. For example, a polyethylene glycol can be derivatized such that one end of the polyethylene glycol contains a SPA and another end contains a SG. In this example, both are activated esters, but the degradation rates of the two linkages are different. For example, a hydrogel prepared with only a PEG-SPA is generally stable at 37 °C for more than about four months, whereas a hydrogel prepared with PEG-SG is often stable for less than about one week. Notably, one hydrogel prepared from PEI and a PEG-SPA/SG having a 60:40 ratio of SPA:SG degraded in about a week.

In certain instance, more than one polyethylene glycol cross-liking agents can be used. For example, a mixture of PEI/PEG-SPA and PEI/PEG-SG. The different cross-linkers may provide linkages having similar or dissimilar degradation rates, and thus the properties of the resulting hydrogel can be controlled.

In certain instances, the polyethylene glycol cross-linking agent contains a hydrophobic moiety. In certain instances, alkyl groups are installed between the polyethylene glycol and the terminal electrophilic groups of the cross-linking agent. In certain instances, the alkyl group contains about 4 to about 30 carbon atoms. In certain instances, the alkyl group contains about 5 to about 15 carbon atoms. In certain instances, the hydrophobic moiety is an aryl or aralkyl group. In certain instances, the alkyl moiety of the aralkyl group contains between 5-10 carbon atoms.

In certain instances, the polyethylene glycol cross-linking agent is represented by the generic formula (i) below, wherein w is an integer in the range of about 5 to 10,000, and n is an integer in the range of about 5 to about 30.

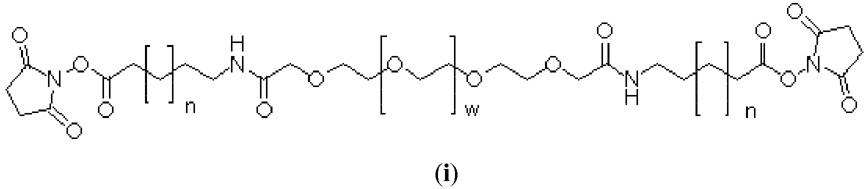

In certain instances, the polyethylene glycol cross-linking agent is represented by the generic formula (ii) below, wherein w is an integer in the range of about 5 to 10,000, and m is an integer in the range of about 1 to about 50.

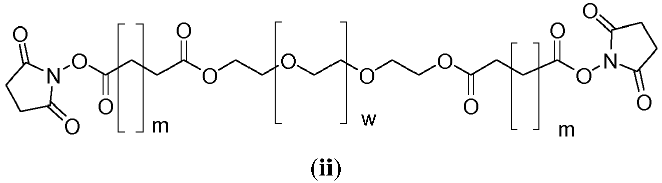

In certain instances the hydrophobic moiety may be used as a foaming agent. The linkages between the polyethylene glycol and the hydrophobic moiety can be esters, amides, carbamates, carbonates, urea, urethane, and so forth.

A further embodiment of the disclosure is an applicator, and methods of use thereof, for chemical peptide ligation reactions, to create a cross-linked gel involving a dendritic polymer. In this reaction an aldehyde, aldehyde-acid or aldehyde-ester reacts with a cysteine-functionalized polymer to form a gel or cross-linked network. In certain instances, the dendritic polymers have nucleophilic groups, such as primary amino groups or thiol groups, which can react with electrophilic groups, such as an acrylate, succinimidyl ester, maleimide, ester aldehyde, or aldehyde on a small molecule. In certain instances, the dendritic polymer has nucleophilic groups capable of reacting with an activated diester of sebacic acid.

Another aspect of the disclosure relates to frothing any of the above-referenced hydrogels. In certain embodiments, the bulk density of the frothed hydrogel is between about 0.1 g/cc and about 1 g/cc, about 0.2 g cc and about 0.9 g/cc, about 0.3 g/cc and 0.8 g/cc, about 0.4 g/cc and about 0.7 g/cc or about 0.45 g/cc and about 0.68 g/cc. In certain embodiments, the bulk density of the frothed hydrogel is between about 0.1 g/cc and about 0.2 g/cc, about 0.2 g/cc and about 0.3 g/cc, about 0.3 g/cc and about 0.4 g/cc, about 0.4 g/cc and about 0.5 g/cc, about 0.5 g/cc and about 0.6 g/cc, about 0.6 g/cc and about 0.7 g/cc, about 0.7 g/cc and about 0.8 g/cc, about 0.8 g/cc and about 0.9 g/cc, or about 0.9 g/cc and about 1.0 g/cc. In certain embodiments, the bu the frothed hydrogel is a frothed polyalkyleneimine hydrogel.

### Selected Applicators

Herein is described an applicator, comprising:
a syringe barrel comprising a sidewall; a separator; a piston; a proximal end; and a distal end; wherein the piston seals the proximal end of the syringe barrel and is slideable within the syringe barrel; the separator divides the syringe barrel into a top compartment, bounded by the piston and the separator, and a bottom compartment, bounded by the distal end of the syringe barrel and the separator; and the distal end is configured to attach to an adapter;
an adapter comprising a removable vial housing portion comprising a piercing structure; a syringe housing portion attached to the distal end of the syringe barrel; a fitting configured to attach to a tip; a valve; a first fluid pathway connecting the bottom compartment to the valve; a second fluid pathway connecting the vial housing portion to the valve; and a third fluid pathway connecting the fitting to the valve; and
a first component in the top compartment.

In certain embodiments, the present invention relates to any one of the aforementioned applicators, further comprising a cap over the fitting.

In certain embodiments, the present invention relates to any one of the aforementioned applicators, further comprising a cap over the piercing structure.

In certain embodiments, the present invention relates to any one of the aforementioned applicators, wherein the first component is a liquid. In certain embodiments, the present invention relates to any one of the aforementioned applicators, wherein the liquid is a buffer. In certain embodiments, the present invention relates to any one of the aforementioned applicators, wherein the liquid is a buffer solution comprising a cross-linking agent.

In certain embodiments, the present invention relates to any one of the aforementioned applicators, wherein the separator is a hydrophobic barrier. In certain embodiments, the present invention relates to any one of the aforementioned applicators, wherein the hydrophobic barrier is a hydrophobic filter.

In certain embodiments, the present invention relates to any one of the aforementioned applicators, wherein instead of a separator, such as a hydrophobic barrier, the applicator comprises a floating plunger and a fluid bypass in the syringe barrel; wherein the floating plunger divides the syringe barrel into top and bottom compartments. The term "fluid bypass" as used herein refers to a structural aspect of, for example, a syringe body, that allows fluid to flow from one compartment to another once a plunger head, or the like, is distally advanced. See, for example, US Patent No. 4,735,616.

In certain embodiments, the present invention relates to any one of the aforementioned applicators, wherein the separator is a two-way valve.

In certain embodiments, the present invention relates to any one of the aforementioned applicators, wherein the separator is a pierceable barrier; and the piston comprises a piercer which can pierce the separator, thereby allowing for fluid communication between the top and bottom compartments.

In certain embodiments, the present invention relates to any one of the aforementioned applicators, wherein the separator is breakable when the syringe barrel is squeezed, thereby allowing for fluid communication between the top and bottom compartments.

In certain embodiments, the present invention relates to any one of the aforementioned applicators, wherein the separator comprises one or more polymers selected from the group consisting of polyacrylics, silicones, polyolefins, polystyrenes, polyesters, polyethers, polyurethanes, polycarbonates, polyamines, or co-polymers thereof. In certain embodiments, the present invention relates to any one of the aforementioned applicators, wherein the separator comprises a metal-containing laminate.

In certain embodiments, the present invention relates to any one of the aforementioned applicators, further comprising a cross-linking agent in the bottom compartment. In certain embodiments, the present invention relates to any one of the aforementioned applicators, wherein the cross-linking agent is a polyalkyleneimine. In certain embodiments, the present invention relates to any one of the aforementioned applicators, wherein the cross-linking agent is PEI.

In certain embodiments, the present invention relates to any one of the aforementioned applicators, further comprising a cross-linking agent in the bottom compartment; and a dissolution aid in the bottom compartment; wherein the cross-linking agent is adsorbed on the dissolution aid. In certain embodiments, the present invention relates to any one of the aforementioned applicators, wherein the dissolution aid is a porous solid. In certain embodiments, the present invention relates to any one of the aforementioned applicators, wherein the dissolution aid is a filter. In certain embodiments, the present invention relates to any one of the aforementioned applicators, wherein the dissolution aid is affixed to the sidewall of the syringe barrel. In certain embodiments, the present invention relates to any one of the aforementioned applicators, wherein the syringe barrel has a retention ring molded into the sidewall; and the retention ring immobilizes the dissolution aid.

In certain embodiments, the present invention relates to any one of the aforementioned applicators, further comprising a filter in the bottom compartment. In certain embodiments, the present invention relates to any one of the aforementioned applicators, wherein the filter is affixed to the sidewall of the syringe barrel. In certain embodiments, the present invention relates to any one of the aforementioned applicators, wherein the syringe barrel has a retention ring molded into the sidewall thereby immobilizing the filter. In certain embodiments, the present invention relates to any one of the aforementioned applicators, wherein the filter knocks down air bubbles.

In certain embodiments, the present invention relates to any one of the aforementioned applicators, wherein the sidewall of the syringe has a step in the bottom compartment (i.e., the syringe barrel has a stepped bore), so that the diameter at the distal end is smaller than the diameter at the proximal end.

In certain embodiments, the present invention relates to any one of the aforementioned applicators, further comprising a retention ring which has a diameter approximately equal to the larger diameter of the stepped bore.

In certain embodiments, the present invention relates to any one of the aforementioned applicators, further comprising a retention ring which has a diameter at its proximal end which is approximately equal to the larger diameter of the stepped bore, and a diameter at its distal end which is approximately equal to the smaller diameter of the stepped bore. In other words, the retention ring can fit over the step in the stepped bore.

In certain embodiments, the present invention relates to any one of the aforementioned applicators, further comprising a dissolution aid or filter; wherein the dissolution aid or filter is held in place at the distal end of the barrel by the retention ring.

In certain embodiments, the present invention relates to any one of the aforementioned applicators, wherein the retention ring is sonically welded in place.

In certain embodiments, the present invention relates to any one of the aforementioned applicators, further comprising a sleeve insert in the distal end of the syringe barrel, thereby creating a stepped bore.

In certain embodiments, the present invention relates to any one of the aforementioned applicators, further comprising a sleeve insert in the distal end of the syringe barrel, thereby creating a stepped bore; wherein the sleeve insert is ultrasonically welded in place.

In certain embodiments, the present invention relates to any one of the aforementioned applicators, further comprising a sleeve insert in the distal end of the syringe barrel, thereby creating a stepped bore; and further comprising a dissolution aid or filter within the interior aspect of the sleeve insert.

In certain embodiments, the present invention relates to any one of the aforementioned applicators, wherein the valve is a three-way valve, which in a first position connects the first and second fluid paths, in a second position connects the first and third fluid paths, and in a third position connects the second and third fluid paths.

In certain embodiments, the present invention relates to any one of the aforementioned applicators, further comprising a vial reversibly attached to the vial housing portion; wherein the vial comprises a second component; the vial is capped by a septum; and the septum is pierced by the piercing structure when the vial is reversibly attached to the vial housing portion, thereby placing the vial in fluid communication with the adapter through the second fluid pathway. In certain embodiments, the present invention relates to any one of the aforementioned applicators, wherein attachment of the vial to the vial housing portion turns the valve so that the vial is in fluid communication with the bottom compartment. In certain embodiments, the present invention relates to any one of the aforementioned applicators, wherein the removal of the vial and the vial housing portion from the adapter turns the valve so that the fitting is in fluid communication with the bottom compartment.

In certain embodiments, the present invention relates to any one of the aforementioned applicators, wherein the second component is a solid. In certain embodiments, the present invention relates to any one of the aforementioned applicators, wherein the solid is an activated PEG. In certain embodiments, the present invention relates to any one of the aforementioned applicators, wherein the activated PEG is a star, dendritic, or branched polymer with between three and less than twenty activating groups. In certain embodiments, the present invention relates to any one of the aforementioned applicators, wherein the solid is 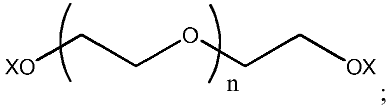 wherein n is 10-200 inclusive; and X is -CH₂C(=O)O(*N*-succinimidyl), -(CH₂)₂C(=O)O(*N*-succinimidyl), -(CH₂)₃C(=O)O(*N*-succinimidyl), -(CH₂)₄C(=O)O(*N*-succinimidyl), -(CH₂)₅C(=O)O(*N*-succinimidyl), -(CH₂)₆C(=O)O(*N*-succinimidyl), -(CH₂)₇C(=O)O(*N*-succinimidyl), -(CH₂)₈C(=O)O(*N*-succinimidyl), -(CH₂)₉C(=O)O(*N*-succinimidyl), -C(=O)CH₂C(=O)O(*N*-succinimidyl), -C(=O)(CH₂)₂C(=O)O(*N*-succinimidyl), -C(=O)(CH₂)₃C(=O)O(*N*-succinimidyl), -C(=O)(CH₂)₄C(=O)O(*N*-succinimidyl), -C(=O)(CH₂)₅C(=O)O(*N*-succinimidyl), -C(=O)(CH₂)₆C(=O)O(*N*-succinimidyl), -C(=O)(CH₂)₇C(=O)O(*N*-succinimidyl), -C(=O)(CH₂)₈C(=O)O(*N*-succinimidyl), or -C(=O)(CH₂)₉C(=O)O(*N*-succinimidyl). In certain embodiments, the present invention relates to any one of the aforementioned applicators, wherein the solid is 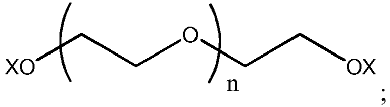 wherein n is 80-120 inclusive; and X is -(CH₂)₃C(=O)O(*N*-succinimidyl), -C(=O)(CH₂)₃C(=O)O(*N*-succinimidyl), or -C(=O)(CH₂)₈C(=O)O(*N*-succinimidyl).

In certain embodiments, the present invention relates to any one of the aforementioned applicators, further comprising a tip attached to the fitting. In certain embodiments, the present invention relates to any one of the aforementioned applicators, wherein the tip is a brush tip, a spray tip, a gas-assisted spray tip, a cannula, a needle, a flexible tube, a bent cannula, a shape-conforming tube, or a combination thereof. In certain embodiments, the present invention relates to any one of the aforementioned applicators, wherein the tip is a blunt cannula. In certain embodiments, the present invention relates to any one of the aforementioned applicators, wherein the fitting is a luer-lock adapter.

In certain embodiments, the present invention relates to any one of the aforementioned applicators, wherein the length of the syringe barrel is between about 1.27 cm (0.5 inches) to about 22.86 cm (9 inches); or about 3.81cm (1.5 inches) to about 10.16 cm (4 inches); or about 5.08 cm (2 inches) to about 7.62 cm (3 inches).

In certain embodiments, the present invention relates to any one of the aforementioned applicators, wherein the diameter of the syringe barrel is between about 0.508 cm (0.2 inches) to about 5.08 cm (2 inches); or about 0.762 cm (0.3 inches) to about 1.905 cm (0.75 inches); or about 1.016 cm (0.4 inches) to about 1.524 cm (0.6 inches).

In certain embodiments, the present invention relates to any one of the aforementioned applicators, wherein the applicator has a sterility assurance level of between about 10⁻³ to about 10^{- 6}.

In certain embodiments, the present invention relates to any one of the aforementioned applicators, wherein the applicator allows for the mixing and delivery of a biodegradable hydrogel.

The present invention is configured so that the applicator comprises two syringe/adapter assemblies connected to the same tip, such as the applicator shown in Figure 3. In certain embodiments, one component can be formulated in each syringe/adapter assembly and then combined in the tip. Herein is described an applicator, comprising:
a first syringe barrel comprising a first sidewall; a first piston; a first proximal end; and a first distal end; wherein the first piston seals the first proximal end of the first syringe barrel and is slideable within the first syringe barrel; and the first distal end is configured to attach to a first adapter;
a second syringe barrel comprising a second sidewall; a second piston; a second proximal end; and a second distal end; wherein the second piston seals the second proximal end of the second syringe barrel and is slideable within the second syringe barrel; and the second distal end is configured to attach to a second adapter;
a first adapter comprising a first removable vial housing portion comprising a first piercing structure; a first syringe housing portion attached to the first distal end of the first syringe barrel; a first fitting configured to attach to a tip; a first valve; a first fluid pathway connecting the first syringe barrel to the first valve; a second fluid pathway connecting the first vial housing portion to the first valve; and a third fluid pathway connecting the first fitting to the first valve;
a second adapter comprising a second removable vial housing portion comprising a second piercing structure; a second syringe housing portion attached to the second distal end of the second syringe barrel; a second fitting configured to attach to a tip; a second valve; a fourth fluid pathway connecting the second syringe barrel to the second valve; a fifth fluid pathway connecting the second vial housing portion to the second valve; and a sixth fluid pathway connecting the second fitting to the second valve;
a tip comprising a first inlet, a second inlet and an outlet, wherein the first inlet is connected to the first fitting, and the second inlet is connected to the second fitting;
a first component in the first top compartment;
a third component in the second top compartment.

In certain embodiments, the present invention relates to any one of the aforementioned applicators, further comprising a cap over the first fitting. In certain embodiments, the present invention relates to any one of the aforementioned applicators, further comprising a cap over the second fitting.

In certain embodiments, the present invention relates to any one of the aforementioned applicators, further comprising a cap over the first piercing structure. In certain embodiments, the present invention relates to any one of the aforementioned applicators, further comprising a cap over the second piercing structure.

In certain embodiments, the present invention relates to any one of the aforementioned applicators, wherein the first component is a liquid. In certain embodiments, the present invention relates to any one of the aforementioned applicators, wherein the liquid is a buffer.

In certain embodiments, the present invention relates to any one of the aforementioned applicators, wherein the third component is a liquid. In certain embodiments, the present invention relates to any one of the aforementioned applicators, wherein the liquid is a buffer.

In certain embodiments, the present invention relates to any one of the aforementioned applicators, further comprising a first filter in the first syringe barrel. In certain embodiments, the present invention relates to any one of the aforementioned applicators, wherein the first filter is affixed to the first sidewall of the first syringe barrel. In certain embodiments, the present invention relates to any one of the aforementioned applicators, wherein the first syringe barrel has a first retention ring molded into the first sidewall thereby immobilizing the first filter. In certain embodiments, the present invention relates to any one of the aforementioned applicators, wherein the first filter knocks down air bubbles.

In certain embodiments, the present invention relates to any one of the aforementioned applicators, further comprising a second filter in the second syringe barrel. In certain embodiments, the present invention relates to any one of the aforementioned applicators, wherein the second filter is affixed to the second sidewall of the second syringe barrel. In certain embodiments, the present invention relates to any one of the aforementioned applicators, wherein the second syringe barrel has a second retention ring molded into the second sidewall thereby immobilizing the second filter. In certain embodiments, the present invention relates to any one of the aforementioned applicators, wherein the second filter knocks down air bubbles.

In certain embodiments, the present invention relates to any one of the aforementioned applicators, wherein the sidewall of the syringe has a step in the bottom compartment (i.e., the first and/or second syringe barrel has a stepped bore), so that the diameter at the distal end is smaller than the diameter at the proximal end.

In certain embodiments, the present invention relates to any one of the aforementioned applicators, further comprising a retention ring which has a diameter approximately equal to the larger diameter of the stepped bore.

In certain embodiments, the present invention relates to any one of the aforementioned applicators, further comprising a retention ring which has a diameter at its proximal end which is approximately equal to the larger diameter of the stepped bore, and a diameter at its distal end which is approximately equal to the smaller diameter of the stepped bore. In other words, the retention ring can fit over the step in the stepped bore.

In certain embodiments, the present invention relates to any one of the aforementioned applicators, further comprising a filter; wherein the filter is held in place at the distal end of the first and/or second syringe barrel by the retention ring.

In certain embodiments, the present invention relates to any one of the aforementioned applicators, wherein the retention ring is sonically welded in place.

In certain embodiments, the present invention relates to any one of the aforementioned applicators, further comprising a sleeve insert in the distal end of the first and/or second syringe barrel, thereby creating a stepped bore.

In certain embodiments, the present invention relates to any one of the aforementioned applicators, further comprising a sleeve insert in the distal end of the first and/or second syringe barrel, thereby creating a stepped bore; wherein the sleeve insert is ultrasonically welded in place.

In certain embodiments, the present invention relates to any one of the aforementioned applicators, further comprising a sleeve insert in the distal end of the first and/or second syringe barrel, thereby creating a stepped bore; and further comprising a filter within the interior aspect of the sleeve insert. In certain embodiments, the present invention relates to any one of the aforementioned applicators, wherein the first valve is a three-way valve, which in a first position connects the first and second fluid paths, in a second position connects the first and third fluid paths, and in a third position connects the second and third fluid paths.

In certain embodiments, the present invention relates to any one of the aforementioned applicators, wherein the second valve is a three-way valve, which in a first position connects the fourth and fifth fluid paths, in a second position connects the fourth and sixth fluid paths, and in a third position connects the fifth and sixth fluid paths.

In certain embodiments, the present invention relates to any one of the aforementioned applicators, further comprising a first vial reversibly attached to the first vial housing portion; wherein the first vial comprises a second component; the first vial is capped by a first septum; and the first septum is pierced by the first piercing structure when the first vial is reversibly attached to the first vial housing portion, thereby placing the first vial in fluid communication with the first adapter through the second fluid pathway. In certain embodiments, the present invention relates to any one of the aforementioned applicators, wherein attachment of the first vial to the first vial housing portion turns the valve so that the first vial is in fluid communication with the first syringe barrel. In certain embodiments, the present invention relates to any one of the aforementioned applicators, wherein attachment of the first vial to the first vial housing portion turns the first valve so that the first vial is in fluid communication with the first syringe barrel. In certain embodiments, the present invention relates to any one of the aforementioned applicators, wherein the removal of the first vial and the first vial housing portion from the adapter turns the first valve so that the first fitting is in fluid communication with the first syringe barrel.

In certain embodiments, the present invention relates to any one of the aforementioned applicators, further comprising a second vial reversibly attached to the second vial housing portion; wherein the second vial comprises a fourth component; the second vial is capped by a second septum; and the second septum is pierced by the second piercing structure when the second vial is reversibly attached to the second vial housing portion, thereby placing the second vial in fluid communication with the second adapter through the fifth fluid pathway. In certain embodiments, the present invention relates to any one of the aforementioned applicators, wherein attachment of the second vial to the second vial housing portion turns the second valve so that the second vial is in fluid communication with the second syringe barrel. In certain embodiments, the present invention relates to any one of the aforementioned applicators, wherein attachment of the second vial to the second vial housing portion turns the second valve so that the second vial is in fluid communication with the second syringe barrel. In certain embodiments, the present invention relates to any one of the aforementioned applicators, wherein the removal of the second vial and the second vial housing portion from the adapter turns the second valve so that the second fitting is in fluid communication with the second syringe barrel.

In certain embodiments, the present invention relates to any one of the aforementioned applicators, wherein the second component is a solid. In certain embodiments, the present invention relates to any one of the aforementioned applicators, wherein the solid is an activated PEG. In certain embodiments, the present invention relates to any one of the aforementioned applicators, wherein the activated PEG is a star, dendritic, or branched polymer with between three and less than twenty activating groups. In certain embodiments, the present invention relates to any one of the aforementioned applicators, wherein the solid is 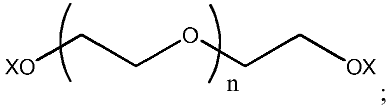 wherein n is 10-200 inclusive; and X is -CH₂C(=O)O(*N*-succinimidyl), -(CH₂)₂C(=O)O(*N*-succinimidyl), -(CH₂)₃C(=O)O(*N*-succinimidyl), -(CH₂)₄C(=O)O(*N*-succinimidyl), -(CH₂)₅C(=O)O(*N*-succinimidyl), -(CH₂)₆C(=O)O(*N*-succinimidyl), -(CH₂)₇C(=O)O(*N*-succinimidyl), -(CH₂)₈C(=O)O(*N*-succinimidyl),-(CH₂)₉C(=O)O(*N*-succinimidyl), -C(=O)CH₂C(=O)O(*N*-succinimidyl), -C(=O)(CH₂)₂C(=O)O(*N*-succinimidyl), -C(=O)(CH₂)₃C(=O)O(*N*-succinimidyl), -C(=O)(CH₂)₄C(=O)O(*N*-succinimidyl), -C(=O)(CH₂)₅C(=O)O(*N*-succinimidyl), -C(=O)(CH₂)₆C(=O)O(*N*-succinimidyl), -C(=O)(CH₂)₇C(=O)O(*N*-succinimidyl), -C(=O)(CH₂)₈C(=O)O(*N*-succinimidyl), or -C(=O)(CH₂)₉C(=O)O(*N*-succinimidyl). In certain embodiments, the present invention relates to any one of the aforementioned applicators, wherein the solid is 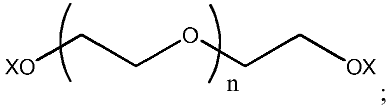 wherein n is 80-120 inclusive; and X is -(CH₂)₃C(=O)O(*N*-succinimidyl), -C(=O)(CH₂)₃C(=O)O(*N*-succinimidyl), or -C(=O)(CH₂)₈C(=O)O(*N*-succinimidyl).

In certain embodiments, the present invention relates to any one of the aforementioned applicators, wherein the fourth component is a cross-linking agent. In certain embodiments, the present invention relates to any one of the aforementioned applicators, wherein the cross-linking agent is a polyalkyleneimine. In certain embodiments, the present invention relates to any one of the aforementioned applicators, wherein the cross-linking agent is PEI.

In certain embodiments, the present invention relates to any one of the aforementioned applicators, further comprising a dissolution aid; wherein the cross-linking agent is adsorbed on the dissolution aid. In certain embodiments, the present invention relates to any one of the aforementioned applicators, wherein the dissolution aid is a porous solid. In certain embodiments, the present invention relates to any one of the aforementioned applicators, wherein the dissolution aid is a filter.

In certain embodiments, the present invention relates to any one of the aforementioned applicators, wherein the length of the first syringe barrel is between about 1.27 cm (0.5 inches) to about 22.86 cm (9 inches); or about 3.81 cm (1.5 inches) to about 10.16 cm (4 inches); or about 5.08 cm (2 inches) to about 7.62 cm (3 inches).

In certain embodiments, the present invention relates to any one of the aforementioned applicators, wherein the length of the second syringe barrel is between about (1.27 cm) 0.5 inches to about 22.86 cm (9 inches); or about 3.81 cm (1.5 inches) to about 10.16 cm (4 inches); or about 5.08 cm (2 inches) to about 7.62 cm (3 inches).

In certain embodiments, the present invention relates to any one of the aforementioned applicators, wherein the diameter of the first syringe barrel is between about 0.508 cm (0.2 inches) to about 5.08 cm (2 inches); or about 0.762 cm (0.3 inches) to about 1.905 cm (0.75 inches); or about 1.016 cm (0.4 inches) to about 1.524 cm (0.6 inches).

In certain embodiments, the present invention relates to any one of the aforementioned applicators, wherein the diameter of the second syringe barrel is between about 0.508 cm (0.2 inches) to about 5.08 cm (2 inches); or about 0.762 cm (0.3 inches) to about 1.905 cm (0.75 inches); or about 1.016 cm (0.4 inches) to about 1.524 cm (0.6 inches).

In certain embodiments, the present invention relates to any one of the aforementioned applicators, wherein the applicator has a sterility assurance level of between about 10⁻³ to about 10^{- 6}.

In certain embodiments, the present invention relates to any one of the aforementioned applicators, wherein the applicator allows for the mixing and delivery of a biodegradable hydrogel.

Herein is described an applicator, comprising:
a syringe barrel comprising a sidewall; a separator; a piston; a proximal end; and a distal end; wherein the piston seals the proximal end of the syringe barrel and is slideable within the syringe barrel; the separator divides the syringe barrel into a top compartment, bounded by the piston and the separator, and a bottom compartment, bounded by the distal end of the syringe barrel and the separator; and the distal end comprises a fitting configured to attach to a tip or cap; and
a first component in the top compartment.

In certain embodiments, the present invention relates to any one of the aforementioned applicators, further comprising a cap at the distal end of the syringe. In certain embodiments, the present invention relates to any one of the aforementioned applicators, wherein the cap at the distal end of the syringe is winged luer cap.

In certain embodiments, the present invention relates to any one of the aforementioned applicators, wherein the first component is a liquid. In certain embodiments, the present invention relates to any one of the aforementioned applicators, wherein the liquid is a buffer. In certain embodiments, the present invention relates to any one of the aforementioned applicators, wherein the liquid is a buffer solution comprising a cross-linking agent.

In certain embodiments, the present invention relates to any one of the aforementioned applicators, wherein the separator is a hydrophobic barrier. In certain embodiments, the present invention relates to any one of the aforementioned applicators, wherein the hydrophobic barrier is a hydrophobic filter.

In certain embodiments, the present invention relates to any one of the aforementioned applicators, wherein instead of a separator, such as a hydrophobic barrier, the applicator comprises a floating plunger and a fluid bypass in the syringe barrel; wherein the floating plunger divides the syringe barrel into top and bottom compartments. The term "fluid bypass" as used herein refers to a structural aspect of, for example, a syringe body, that allows fluid to flow from one compartment to another once a plunger head, or the like, is distally advanced. See, for example, US Patent No. 4,735,616.

In certain embodiments, the present invention relates to any one of the aforementioned applicators, wherein the separator is a two-way valve.

In certain embodiments, the present invention relates to any one of the aforementioned applicators, wherein the separator is a pierceable barrier; and the piston comprises a piercer which can pierce the separator, thereby allowing for fluid communication between the top and bottom compartments.

In certain embodiments, the present invention relates to any one of the aforementioned applicators, wherein the separator is breakable when the syringe barrel is squeezed, thereby allowing for fluid communication between the top and bottom compartments.

In certain embodiments, the present invention relates to any one of the aforementioned applicators, wherein the separator comprises one or more polymers selected from the group consisting of polyacrylics, silicones, polyolefins, polystyrenes, polyesters, polyethers, polyurethanes, polycarbonates, polyamines, or co-polymers thereof. In certain embodiments, the present invention relates to any one of the aforementioned applicators, wherein the separator comprises a metal-containing laminate.

In certain embodiments, the present invention relates to any one of the aforementioned applicators, further comprising a cross-linking agent in the bottom compartment. In certain embodiments, the present invention relates to any one of the aforementioned applicators, wherein the cross-linking agent is a polyalkyleneimine. In certain embodiments, the present invention relates to any one of the aforementioned applicators, wherein the cross-linking agent is PEI.

In certain embodiments, the present invention relates to any one of the aforementioned applicators, further comprising a cross-linking agent in the bottom compartment; and a dissolution aid in the bottom compartment; wherein the cross-linking agent is adsorbed on the dissolution aid. In certain embodiments, the present invention relates to any one of the aforementioned applicators, wherein the dissolution aid is a porous solid. In certain embodiments, the present invention relates to any one of the aforementioned applicators, wherein the dissolution aid is a filter. In certain embodiments, the present invention relates to any one of the aforementioned applicators, wherein the dissolution aid is affixed to the sidewall of the syringe barrel. In certain embodiments, the present invention relates to any one of the aforementioned applicators, wherein the syringe barrel has a retention ring molded into the sidewall; and the retention ring immobilizes the dissolution aid.

In certain embodiments, the present invention relates to any one of the aforementioned applicators, further comprising a filter in the bottom compartment. In certain embodiments, the present invention relates to any one of the aforementioned applicators, wherein the filter is affixed to the sidewall of the syringe barrel. In certain embodiments, the present invention relates to any one of the aforementioned applicators, wherein the syringe barrel has a retention ring molded into the sidewall thereby immobilizing the filter. In certain embodiments, the present invention relates to any one of the aforementioned applicators, wherein the filter knocks down air bubbles.

In certain embodiments, the present invention relates to any one of the aforementioned applicators, wherein the sidewall of the syringe has a step in the bottom compartment (i.e., the syringe barrel has a stepped bore), so that the diameter at the distal end is smaller than the diameter at the proximal end.

In certain embodiments, the present invention relates to any one of the aforementioned applicators, further comprising a retention ring which has a diameter approximately equal to the larger diameter of the stepped bore.

In certain embodiments, the present invention relates to any one of the aforementioned applicators, further comprising a retention ring which has a diameter at its proximal end which is approximately equal to the larger diameter of the stepped bore, and a diameter at its distal end which is approximately equal to the smaller diameter of the stepped bore. In other words, the retention ring can fit over the step in the stepped bore.

In certain embodiments, the present invention relates to any one of the aforementioned applicators, further comprising a dissolution aid or filter; wherein the dissolution aid or filter is held in place at the distal end of the barrel by the retention ring.

In certain embodiments, the present invention relates to any one of the aforementioned applicators, wherein the retention ring is sonically welded in place.

In certain embodiments, the present invention relates to any one of the aforementioned applicators, further comprising a sleeve insert in the distal end of the syringe barrel, thereby creating a stepped bore.

In certain embodiments, the present invention relates to any one of the aforementioned applicators, further comprising a sleeve insert in the distal end of the syringe barrel, thereby creating a stepped bore; wherein the sleeve insert is ultrasonically welded in place.

In certain embodiments, the present invention relates to any one of the aforementioned applicators, further comprising a sleeve insert in the distal end of the syringe barrel, thereby creating a stepped bore; and further comprising a dissolution aid or filter within the interior aspect of the sleeve insert.

In certain embodiments, the present invention relates to any one of the aforementioned applicators, further comprising a tip attached to the fitting. In certain embodiments, the present invention relates to any one of the aforementioned applicators, wherein the tip is a brush tip, a spray tip, a gas-assisted spray tip, a cannula, a needle, a flexible tube, a bent cannula, a shape-conforming tube, or a combination thereof. In certain embodiments, the present invention relates to any one of the aforementioned applicators, wherein the tip is a blunt cannula. In certain embodiments, the present invention relates to any one of the aforementioned applicators, wherein the fitting is a luer-lock adapter.

In certain embodiments, the present invention relates to any one of the aforementioned applicators, wherein the length of the syringe barrel is between about 1.27 cm (0.5 inches) to about 22.86 cm (9 inches); or about 3.81 cm (1.5 inches) to about 10.16 cm (4 inches); or about 5.08 cm (2 inches) to about 7.62 cm (3 inches).

In certain embodiments, the present invention relates to any one of the aforementioned applicators, wherein the diameter of the syringe barrel is between about 0.508 cm (0.2 inches) to about 5.08 cm (2 inches); or about 0.762 cm (0.3 inches) to about 1.905 cm (0.75 inches); or about 1.016 cm (0.4 inches) to about 1.524 cm (0.6 inches).

In certain embodiments, the present invention relates to any one of the aforementioned applicators, wherein the applicator has a sterility assurance level of between about 10⁻³ to about 10^{- 6}.

In certain embodiments, the present invention relates to any one of the aforementioned applicators, wherein the applicator allows for the mixing and delivery of a biodegradable hydrogel.

### Selected Vials

Another aspect of the invention relates to a vial capped by a septum, wherein the septum has a slit through which a tip can pass; and a second component in the vial.

In certain embodiments, the present invention relates to any one of the aforementioned applicators, wherein the slit has a cruciate shape.

In certain embodiments, the present invention relates to any one of the aforementioned applicators, wherein the second component is a solid. In certain embodiments, the present invention relates to any one of the aforementioned applicators, wherein the solid is an activated PEG. In certain embodiments, the present invention relates to any one of the aforementioned applicators, wherein the activated PEG is a star, dendritic, or branched polymer with between three and less than twenty activating groups. In certain embodiments, the present invention relates to any one of the aforementioned applicators, wherein the solid is 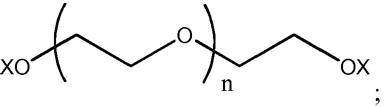 wherein n is 10-200 inclusive; and X is -CH₂C(=O)O(*N*-succinimidyl), -(CH₂)₂C(=O)O(*N*-succinimidyl), -(CH₂)₃C(=O)O(*N*-succinimidyl), -(CH₂)₄C(=O)O(*N*-succinimidyl), -(CH₂)₅C(=O)O(*N*-succinimidyl), -(CH₂)₆C(=O)O(*N*-succinimidyl), -(CH₂)₇C(=O)O(*N*-succinimidyl), -(CH₂)₈C(=O)O(*N*-succinimidyl),-(CH₂)₉C(=O)O(*N*-succinimidyl), -C(=O)CH₂C(=O)O(*N*-succinimidyl), -C(=O)(CH₂)₂C(=O)O(*N*-succinimidyl), -C(=O)(CH₂)₃C(=O)O(*N*-succinimidyl), -C(=O)(CH₂)₄C(=O)O(*N*-succinimidyl), -C(=O)(CH₂)₅C(=O)O(*N*-succinimidyl), -C(=O)(CH₂)₆C(=O)O(*N*-succinimidyl), -C(=O)(CH₂)₇C(=O)O(*N*-succinimidyl), -C(=O)(CH₂)₈C(=O)O(*N*-succinimidyl), or -C(=O)(CH₂)₉C(=O)O(*N*-succinimidyl). In certain embodiments, the present invention relates to any one of the aforementioned applicators, wherein the solid is 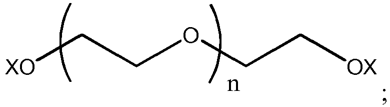 wherein n is 80-120 inclusive; and X is -(CH₂)₃C(=O)O(*N*-succinimidyl), -C(=O)(CH₂)₃C(=O)O(*N*-succinimidyl), or -C(=O)(CH₂)₈C(=O)O(*N*-succinimidyl).

In certain embodiments, the present invention relates to any one of the aforementioned applicators, wherein the tip is a blunt cannula.

### Selected Methods

Herein is described a method of applying a composition to a surface via an applicator; wherein the applicator comprises (1) a syringe barrel comprising a sidewall; a separator; a piston; a proximal end; and a distal end; wherein the piston seals the proximal end of the syringe barrel and is slideable within the syringe barrel; the separator divides the syringe barrel into a top compartment, bounded by the piston and the separator, and a bottom compartment, bounded by the distal end of the syringe barrel and the separator; and the distal end is configured to attach to an adapter; (2) an adapter comprising a removable vial housing portion comprising a piercing structure; a syringe housing portion attached to the distal end of the syringe barrel; a fitting configured to attach to a tip; a valve; a first fluid pathway connecting the bottom compartment to the valve; a second fluid pathway connecting the vial housing portion to the valve; and a third fluid pathway connecting the fitting to the valve; and (3) a first component in the top compartment;
comprising the steps of:
attaching a tip to the fitting;
attaching a vial to the vial housing portion of the adapter; wherein the vial comprises a second component; the vial is capped by a septum; and the septum is pierced by the piercing structure when the vial is attached to the vial housing portion;
advancing the piston towards the proximal end of the syringe barrel, thereby forcing the first component into the bottom chamber, through the adapter, through the piercing structure, and into the vial, thereby forming a mixture;
optionally retracting and advancing the piston at least once to assist in the mixing of the first component and the second component;
retracting the piston, thereby pulling the mixture into the bottom compartment;
removing the vial housing portion of the adapter, wherein the vial is attached;
advancing the piston, thereby forcing the mixture through the adapter and the tip, thereby applying the mixture to the surface.

In certain embodiments, the present invention relates to any one of the aforementioned methods, wherein the mixture gels on the surface to form a biodegradable hydrogel.

In certain embodiments, the present invention relates to any one of the aforementioned methods, further comprising the step of agitating the applicator to promote mixing of the mixture.

In certain embodiments, the present disclosure relates to any one of the aforementioned methods, wherein the piston is retracting and advancing at least once to assist in the mixing of the first component and the second component.

In certain embodiments, the present disclosure relates to any one of the aforementioned methods, wherein the piston is retracted and advanced at least once to assist in the mixing of the first component and the second component; thereby introducing air into the mixture (i.e., frothing the mixture).

In certain embodiments, the present disclosure relates to any one of the aforementioned methods, wherein the applicator further comprises a cap over the fitting; and the method further comprises removing the cap over the fitting before the tip is attached.

In certain embodiments, the present disclosure relates to any one of the aforementioned methods, wherein the applicator further comprises a cap over the piercing structure; and the method further comprises removing the cap over the piercing structure before the vial is attached.

In certain embodiments, the present disclosure relates to any one of the aforementioned methods, wherein the first component is a liquid. In certain embodiments, the present disclosure relates to any one of the aforementioned methods, wherein the liquid is a buffer. In certain embodiments, the present disclosure relates to any one of the aforementioned methods, wherein the liquid is a buffer solution comprising a cross-linking agent.

In certain embodiments, the present disclosure relates to any one of the aforementioned methods, wherein the separator is a hydrophobic barrier. In certain embodiments, the present disclosure relates to any one of the aforementioned methods, wherein the hydrophobic barrier is a hydrophobic filter.

In certain embodiments, the present disclosure relates to any one of the aforementioned methods, wherein instead of a separator, such as a hydrophobic barrier, the applicator comprises a floating plunger and a fluid bypass in the syringe barrel; wherein the floating plunger divides the syringe barrel into top and bottom compartments. The term "fluid bypass" as used herein refers to a structural aspect of, for example, a syringe body, that allows fluid to flow from one compartment to another once a plunger head, or the like, is distally advanced. See, for example, US Patent No. 4,735,616.

In certain embodiments, the present disclosure relates to any one of the aforementioned methods, wherein the separator is a two-way valve; and the valve is opened to allow for fluid communication between the top and bottom compartments before the piston is advanced.

In certain embodiments, the present disclosure relates to any one of the aforementioned methods, wherein the separator is a pierceable barrier; and the piston comprises a piercer which can pierce the separator, thereby allowing for fluid communication between the top and bottom compartments.

In certain embodiments, the present disclosure relates to any one of the aforementioned methods, wherein the separator is breakable when the syringe barrel is squeezed, thereby allowing for fluid communication between the top and bottom compartments; and the method further comprises the step of breaking the separator thereby placing the top and bottom compartments in fluid communication before advancing the piston.

In certain embodiments, the present disclosure relates to any one of the aforementioned methods, wherein the separator comprises one or more polymers selected from the group consisting of polyacrylics, silicones, polyolefins, polystyrenes, polyesters, polyethers, polyurethanes, polycarbonates, polyamines, or co-polymers thereof. In certain embodiments, the present disclosure relates to any one of the aforementioned methods, wherein the separator comprises a metal-containing laminate.

In certain embodiments, the present disclosure relates to any one of the aforementioned methods, wherein the applicator further comprises a cross-linking agent in the bottom compartment. In certain embodiments, the present disclosure relates to any one of the aforementioned methods, wherein the cross-linking agent is a polyalkyleneimine. In certain embodiments, the present disclosure relates to any one of the aforementioned methods, wherein the cross-linking agent is PEI.

In certain embodiments, the present disclosure relates to any one of the aforementioned methods, wherein the applicator further comprises a cross-linking agent in the bottom compartment; and a dissolution aid in the bottom compartment; wherein the cross-linking agent is adsorbed on the dissolution aid. In certain embodiments, the present disclosure relates to any one of the aforementioned methods, wherein the dissolution aid is a porous solid. In certain embodiments, the present disclosure relates to any one of the aforementioned methods, wherein the dissolution aid is a filter. In certain embodiments, the present disclosure relates to any one of the aforementioned methods, wherein the dissolution aid is affixed to the sidewall of the syringe barrel. In certain embodiments, the present disclosure relates to any one of the aforementioned methods, wherein the syringe barrel has a retention ring molded into the sidewall; and the retention ring immobilizes the dissolution aid.

In certain embodiments, the present disclosure relates to any one of the aforementioned methods, wherein the applicator further comprises a filter in the bottom compartment. In certain embodiments, the present disclosure relates to any one of the aforementioned methods, wherein the filter is affixed to the sidewall of the syringe barrel. In certain embodiments, the present disclosure relates to any one of the aforementioned methods, wherein the syringe barrel has a retention ring molded into the sidewall thereby immobilizing the filter. In certain embodiments, the present disclosure relates to any one of the aforementioned methods, wherein the filter knocks down air bubbles.

In certain embodiments, the present disclosure relates to any one of the aforementioned methods, wherein the sidewall of the syringe has a step in the bottom compartment (i.e., the syringe barrel has a stepped bore), so that the diameter at the distal end is smaller than the diameter at the proximal end.

In certain embodiments, the present disclosure relates to any one of the aforementioned methods, further comprising a retention ring which has a diameter approximately equal to the larger diameter of the stepped bore.

In certain embodiments, the present disclosure relates to any one of the aforementioned methods, further comprising a retention ring which has a diameter at its proximal end which is approximately equal to the larger diameter of the stepped bore, and a diameter at its distal end which is approximately equal to the smaller diameter of the stepped bore. In other words, the retention ring can fit over the step in the stepped bore.

In certain embodiments, the present disclosure relates to any one of the aforementioned methods, further comprising a dissolution aid or filter; wherein the dissolution aid or filter is held in place at the distal end of the barrel by the retention ring.

In certain embodiments, the present disclosure relates to any one of the aforementioned methods, wherein the retention ring is sonically welded in place.

In certain embodiments, the present disclosure relates to any one of the aforementioned methods, further comprising a sleeve insert in the distal end of the syringe barrel, thereby creating a stepped bore.

In certain embodiments, the present disclosure relates to any one of the aforementioned methods, further comprising a sleeve insert in the distal end of the syringe barrel, thereby creating a stepped bore; wherein the sleeve insert is ultrasonically welded in place.

In certain embodiments, the present disclosure relates to any one of the aforementioned methods, further comprising a sleeve insert in the distal end of the syringe barrel, thereby creating a stepped bore; and further comprising a dissolution aid or filter within the interior aspect of the sleeve insert.

In certain embodiments, the present disclosure relates to any one of the aforementioned methods, wherein the valve is a three-way valve, which in a first position connects the first and second fluid paths, in a second position connects the first and third fluid paths, and in a third position connects the second and third fluid paths.

In certain embodiments, the present disclosure relates to any one of the aforementioned methods, wherein attachment of the vial to the vial housing portion turns the valve so that the vial is in fluid communication with the bottom compartment. In certain embodiments, the present disclosure relates to any one of the aforementioned methods, wherein the removal of the vial and the vial housing portion from the adapter turns the valve so that the fitting is in fluid communication with the bottom compartment.

In certain embodiments, the present disclosure relates to any one of the aforementioned methods, wherein the second component is a solid. In certain embodiments, the present disclosure relates to any one of the aforementioned methods, wherein the solid is an activated PEG. In certain embodiments, the present disclosure relates to any one of the aforementioned methods, wherein the activated PEG is a star, dendritic, or branched polymer with between three and less than twenty activating groups. In certain embodiments, the present disclosure relates to any one of the aforementioned methods, wherein the solid is 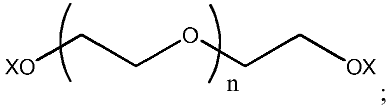 wherein n is 10-200 inclusive; and X is -CH₂C(=O)O(*N*-succinimidyl), -(CH₂)₂C(=O)O(*N*-succinimidyl), -(CH₂)₃C(=O)O(*N*-succinimidyl), -(CH₂)₄C(=O)O(*N*-succinimidyl), -(CH₂)₅C(=O)O(*N*-succinimidyl), -(CH₂)₆C(=O)O(*N*-succinimidyl), -(CH₂)₇C(=O)O(*N*-succinimidyl), -(CH₂)₈C(=O)O(*N*-succinimidyl), -(CH₂)₉C(=O)O(*N*-succinimidyl), -C(=O)CH₂C(=O)O(*N*-succinimidyl), -C(=O)(CH₂)₂C(=O)O(*N*-succinimidyl), -C(=O)(CH₂)₃C(=O)O(*N*-succinimidyl), -C(=O)(CH₂)₄C(=O)O(*N*-succinimidyl), -C(=O)(CH₂)₅C(=O)O(*N*-succinimidyl), -C(=O)(CH₂)₆C(=O)O(*N*-succinimidyl), -C(=O)(CH₂)₇C(=O)O(*N*-succinimidyl), -C(=O)(CH₂)₈C(=O)O(*N*-succinimidyl), or -C(=O)(CH₂)₉C(=O)O(*N*-succinimidyl). In certain embodiments, the present disclosure relates to any one of the aforementioned methods, wherein the solid is 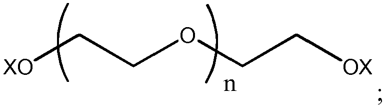 wherein n is 80-120 inclusive; and X is -(CH₂)₃C(=O)O(*N*-succinimidyl), -C(=O)(CH₂)₃C(=O)O(*N*-succinimidyl), or -C(=O)(CH₂)₈C(=O)O(*N*-succinimidyl).

In certain embodiments, the present disclosure relates to any one of the aforementioned methods, wherein the tip is a brush tip, a spray tip, a gas-assisted spray tip, a cannula, a needle, a flexible tube, a bent cannula, a shape-conforming tube, or a combination thereof. In certain embodiments, the present disclosure relates to any one of the aforementioned methods, wherein the tip is a blunt cannula. In certain embodiments, the present disclosure relates to any one of the aforementioned methods, wherein the fitting is a luer-lock adapter.

In certain embodiments, the present disclosure relates to any one of the aforementioned methods, wherein the length of the syringe barrel is between about 1.27 cm (0.5 inches) to about 22.86 cm (9 inches); or about 3.81 cm (1.5 inches) to about 10.16 cm (4 inches); or about 5.08 cm (2 inches) to about 7.62 cm (3 inches).

In certain embodiments, the present disclosure relates to any one of the aforementioned methods, wherein the diameter of the syringe barrel is between about 0.508 cm (0.2 inches) to about 5.08 cm (2 inches); or about 0.762 cm (0.3 inches) to about 1.905 cm (0.75 inches); or about 1.016 cm (0.4 inches) to about 1.524 cm (0.6 inches).

Herein is described a method of applying a composition to a surface via an applicator; wherein the applicator comprises (1) a syringe barrel comprising a sidewall; a separator; a piston; a proximal end; and a distal end; wherein the piston seals the proximal end of the syringe barrel and is slideable within the syringe barrel; the separator divides the syringe barrel into a top compartment, bounded by the piston and the separator, and a bottom compartment, bounded by the distal end of the syringe barrel and the separator; and the distal end is configured to attach to an adapter; (2) a cap covering the distal end of the syringe barrel; (3) a first component in the top compartment; and (4) a third component in the bottom compartment;
comprising the steps of:
optionally holding the syringe barrel so that the distal end is pointing up;
advancing the piston towards the proximal end of the syringe barrel, thereby forcing the first component into the bottom chamber, thereby forming a first mixture of the first and the third components;
optionally retracting and advancing the piston at least once to assist in the mixing of the first mixture;
removing the cap;
attaching an adapter to the distal end of the syringe barrel, wherein the adaptor comprises a removable vial housing portion comprising a piercing structure; a syringe housing portion attached to the distal end of the syringe barrel; a fitting configured to attach to a tip; a valve; a first fluid pathway connecting the bottom compartment to the valve; a second fluid pathway connecting the vial housing portion to the valve; and a third fluid pathway connecting the fitting to the valve;
attaching a tip to the fitting;
attaching a vial to the vial housing portion of the adapter; wherein the vial comprises a second component; the vial is capped by a septum; and the septum is pierced by the piercing structure when the vial is attached to the vial housing portion;
advancing the piston towards the proximal end of the syringe barrel, thereby forcing the first mixture into the bottom chamber, through the adapter, through the piercing structure, and into the vial, thereby forming a second mixture;
optionally retracting and advancing the piston at least once to assist in the mixing of the second mixture;
retracting the piston, thereby pulling the second mixture into the bottom compartment;
removing the vial housing portion of the adapter, wherein the vial is attached;
advancing the piston, thereby forcing the second mixture through the adapter and the tip, thereby applying the second mixture to the surface.

In certain embodiments, the present disclosure relates to any one of the aforementioned methods, wherein the second mixture gels on the surface to form a biodegradable hydrogel.

In certain embodiments, the present disclosure relates to any one of the aforementioned methods, further comprising the step of agitating the applicator to promote mixing of the first and/or second mixture.

In certain embodiments, the present disclosure relates to any one of the aforementioned methods, wherein the piston is retracted and advanced at least once to assist in the mixing of the first mixture.

In certain embodiments, the present disclosure relates to any one of the aforementioned methods, wherein the piston is retracted and advanced at least once to assist in the mixing of the first mixture; thereby introducing air into the first mixture (i.e., frothing the mixture).

In certain embodiments, the present disclosure relates to any one of the aforementioned methods, wherein the piston is retracted and advanced at least once to assist in the mixing of the second mixture.

In certain embodiments, the present disclosure relates to any one of the aforementioned methods, wherein the piston is retracted and advanced at least once to assist in the mixing of the second mixture; thereby introducing air into the second mixture (i.e., frothing the mixture).

In certain embodiments, the present disclosure relates to any one of the aforementioned methods, wherein the applicator further comprises a cap over the fitting; and the method further comprises removing the cap over the fitting before the tip is attached.

In certain embodiments, the present disclosure relates to any one of the aforementioned methods, wherein the applicator further comprises a cap over the piercing structure; and the method further comprises removing the cap over the piercing structure before the vial is attached.

In certain embodiments, the present disclosure relates to any one of the aforementioned methods, wherein the first component is a liquid. In certain embodiments, the present disclosure relates to any one of the aforementioned methods, wherein the liquid is a buffer. In certain embodiments, the present disclosure relates to any one of the aforementioned methods, wherein the liquid is a buffer solution comprising a cross-linking agent.

In certain embodiments, the present disclosure relates to any one of the aforementioned methods, wherein the separator is a hydrophobic barrier. In certain embodiments, the present disclosure relates to any one of the aforementioned methods, wherein the hydrophobic barrier is a hydrophobic filter.

In certain embodiments, the present disclosure relates to any one of the aforementioned methods, wherein instead of a separator, such as a hydrophobic barrier, the applicator comprises a floating plunger and a fluid bypass in the syringe barrel; wherein the floating plunger divides the syringe barrel into top and bottom compartments. The term "fluid bypass" as used herein refers to a structural aspect of, for example, a syringe body, that allows fluid to flow from one compartment to another once a plunger head, or the like, is distally advanced. See, for example, US Patent No. 4,735,616.

In certain embodiments, the present disclosure relates to any one of the aforementioned methods, wherein the separator is a two-way valve; and the valve is opened to allow for fluid communication between the top and bottom compartments before the piston is advanced.

In certain embodiments, the present disclosure relates to any one of the aforementioned methods, wherein the separator is a pierceable barrier; and the piston comprises a piercer which can pierce the separator, thereby allowing for fluid communication between the top and bottom compartments.

In certain embodiments, the present disclosure relates to any one of the aforementioned methods, wherein the separator is breakable when the syringe barrel is squeezed, thereby allowing for fluid communication between the top and bottom compartments; and the method further comprises the step of breaking the separator thereby placing the top and bottom compartments in fluid communication before advancing the piston.

In certain embodiments, the present disclosure relates to any one of the aforementioned methods, wherein the separator comprises one or more polymers selected from the group consisting of polyacrylics, silicones, polyolefins, polystyrenes, polyesters, polyethers, polyurethanes, polycarbonates, polyamines, or co-polymers thereof. In certain embodiments, the present disclosure relates to any one of the aforementioned methods, wherein the separator comprises a metal-containing laminate.

In certain embodiments, the present disclosure relates to any one of the aforementioned methods, wherein the third component is a cross-linking agent. In certain embodiments, the present disclosure relates to any one of the aforementioned methods, wherein the cross-linking agent is a polyalkyleneimine. In certain embodiments, the present disclosure relates to any one of the aforementioned methods, wherein the cross-linking agent is PEI.

In certain embodiments, the present disclosure relates to any one of the aforementioned methods, wherein the applicator further comprises a dissolution aid in the bottom compartment; wherein the cross-linking agent is adsorbed on the dissolution aid. In certain embodiments, the present disclosure relates to any one of the aforementioned methods, wherein the dissolution aid is a porous solid. In certain embodiments, the present disclosure relates to any one of the aforementioned methods, wherein the dissolution aid is a filter. In certain embodiments, the present disclosure relates to any one of the aforementioned methods, wherein the dissolution aid is affixed to the sidewall of the syringe barrel. In certain embodiments, the present disclosure relates to any one of the aforementioned methods, wherein the syringe barrel has a retention ring molded into the sidewall; and the retention ring immobilizes the dissolution aid.

In certain embodiments, the present disclosure relates to any one of the aforementioned methods, wherein the applicator further comprises a filter in the bottom compartment. In certain embodiments, the present disclosure relates to any one of the aforementioned methods, wherein the filter is affixed to the sidewall of the syringe barrel. In certain embodiments, the present disclosure relates to any one of the aforementioned methods, wherein the syringe barrel has a retention ring molded into the sidewall thereby immobilizing the filter. In certain embodiments, the present disclosure relates to any one of the aforementioned methods, wherein the filter knocks down air bubbles.

In certain embodiments, the present disclosure relates to any one of the aforementioned methods, wherein the sidewall of the syringe has a step in the bottom compartment (i.e., the syringe barrel has a stepped bore), so that the diameter at the distal end is smaller than the diameter at the proximal end.

In certain embodiments, the present disclosure relates to any one of the aforementioned methods, further comprising a retention ring which has a diameter approximately equal to the larger diameter of the stepped bore.

In certain embodiments, the present disclosure relates to any one of the aforementioned methods, further comprising a retention ring which has a diameter at its proximal end which is approximately equal to the larger diameter of the stepped bore, and a diameter at its distal end which is approximately equal to the smaller diameter of the stepped bore. In other words, the retention ring can fit over the step in the stepped bore.

In certain embodiments, the present disclosure relates to any one of the aforementioned methods, further comprising a dissolution aid or filter; wherein the dissolution aid or filter is held in place at the distal end of the barrel by the retention ring.

In certain embodiments, the present disclosure relates to any one of the aforementioned methods, wherein the retention ring is sonically welded in place.

In certain embodiments, the present disclosure relates to any one of the aforementioned methods, further comprising a sleeve insert in the distal end of the syringe barrel, thereby creating a stepped bore.

In certain embodiments, the present disclosure relates to any one of the aforementioned methods, further comprising a sleeve insert in the distal end of the syringe barrel, thereby creating a stepped bore; wherein the sleeve insert is ultrasonically welded in place.

In certain embodiments, the present disclosure relates to any one of the aforementioned methods, further comprising a sleeve insert in the distal end of the syringe barrel, thereby creating a stepped bore; and further comprising a dissolution aid or filter within the interior aspect of the sleeve insert.

In certain embodiments, the present disclosure relates to any one of the aforementioned methods, wherein the valve is a three-way valve, which in a first position connects the first and second fluid paths, in a second position connects the first and third fluid paths, and in a third position connects the second and third fluid paths.

In certain embodiments, the present disclosure relates to any one of the aforementioned methods, wherein attachment of the vial to the vial housing portion turns the valve so that the vial is in fluid communication with the bottom compartment. In certain embodiments, the present invention relates to any one of the aforementioned applicators. In certain embodiments, the present invention relates to any one of the aforementioned applicators, wherein the removal of the vial and the vial housing portion from the adapter turns the valve so that the fitting is in fluid communication with the bottom compartment.

In certain embodiments, the present disclosure relates to any one of the aforementioned methods, wherein the second component is a solid. In certain embodiments, the present disclosure relates to any one of the aforementioned methods, wherein the solid is an activated PEG. In certain embodiments, the present disclosure relates to any one of the aforementioned methods, wherein the activated PEG is a star, dendritic, or branched polymer with between three and less than twenty activating groups. In certain embodiments, the present disclosure relates to any one of the aforementioned methods, wherein the solid is 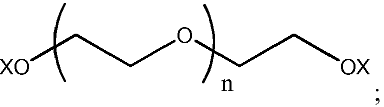 wherein n is 10-200 inclusive; and X is -CH₂C(=O)O(*N*-succinimidyl), -(CH₂)₂C(=O)O(*N*-succinimidyl), -(CH₂)₃C(=O)O(*N*-succinimidyl), -(CH₂)₄C(=O)O(*N*-succinimidyl), -(CH₂)₅C(=O)O(*N*-succinimidyl), -(CH₂)₆C(=O)O(*N*-succinimidyl), -(CH₂)₇C(=O)O(N-succinimidyl), -(CH₂)₈C(=O)O(*N*-succinimidyl), -(CH₂)₉C(=O)O(*N*-succinimidyl), -C(=O)CH₂C(=O)O(*N*-succinimidyl), -C(=O)(CH₂)₂C(=O)O(*N*-succinimidyl), -C(=O)(CH₂)₃C(=O)O(*N*-succinimidyl), -C(=O)(CH₂)₄C(=O)O(*N*-succinimidyl), -C(=O)(CH₂)₅C(=O)O(*N*-succinimidyl), -C(=O)(CH₂)₆C(=O)O(*N*-succinimidyl), -C(=O)(CH₂)₇C(=O)O(*N*-succinimidyl), -C(=O)(CH₂)₈C(=O)O(*N*-succinimidyl), or -C(=O)(CH₂)₉C(=O)O(*N*-succinimidyl). In certain embodiments, the present disclosure relates to any one of the aforementioned methods, wherein the solid is 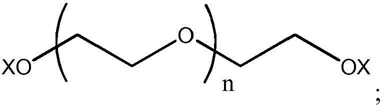 wherein n is 80-120 inclusive; and X is -(CH₂)₃C(=O)O(*N*-succinimidyl), -C(=O)(CH₂)₃C(=O)O(*N*-succinimidyl), or -C(=O)(CH₂)₈C(=O)O(*N*-succinimidyl).

In certain embodiments, the present disclosure relates to any one of the aforementioned methods, wherein the tip is a brush tip, a spray tip, a gas-assisted spray tip, a cannula, a needle, a flexible tube, a bent cannula, a shape-conforming tube, or a combination thereof. In certain embodiments, the present disclosure relates to any one of the aforementioned methods, wherein the tip is a blunt cannula. In certain embodiments, the present disclosure relates to any one of the aforementioned methods, wherein the fitting is a luer-lock adapter.

In certain embodiments, the present disclosure relates to any one of the aforementioned methods, wherein the length of the syringe barrel is between about 1.27 cm (0.5 inches) to about 22.86 cm (9 inches); or about 3.81 cm (1.5 inches) to about 10.16 cm (4 inches); or about 5.08 cm (2 inches) to about 7.62 cm (3 inches).

In certain embodiments, the present disclosure relates to any one of the aforementioned methods, wherein the diameter of the syringe barrel is between about 0.508 cm (0.2 inches) to about 5.08 cm (2 inches); or about 0.762 cm (0.3 inches) to about 1.905 cm (0.75 inches); or about 1.016 cm (0.4 inches) to about 1.524 cm (0.6 inches).

In certain embodiments, the present disclosure relates to any one of the aforementioned methods, where any one of the aforementioned double-barreled applicators, such as those described herein, is used instead of the single barrel apparatus described above. In certain embodiments, the first and second components, and third and fourth components, can be reconstituted at the same time or at different times, as long as all the components are combined in the tip at the same time, prior to being placed on the surface.

Herein is described a method of applying a composition to a surface via an applicator; wherein the applicator comprises (1) a syringe barrel comprising a sidewall; a separator; a piston; a proximal end; and a distal end; wherein the piston seals the proximal end of the syringe barrel and is slideable within the syringe barrel; the separator divides the syringe barrel into a top compartment, bounded by the piston and the separator, and a bottom compartment, bounded by the distal end of the syringe barrel and the separator; and the distal end comprises a fitting configured to attach to a tip; (2) a first component in the top compartment; and (3) a tip affixed to the fitting;
comprising the steps of:
inserting the tip into a vial; wherein the vial is capped by a septum, the septum has a slit through which the tip is inserted; and there is a second component in the vial;
advancing the piston towards the proximal end of the syringe barrel, thereby forcing the first component into the bottom chamber, through the tip, and into the vial, thereby forming a mixture;
retracting the piston, thereby pulling the mixture into the bottom compartment;
removing the tip from the vial; and
advancing the piston, thereby forcing the mixture through the tip, thereby applying the mixture to the surface.

In certain embodiments, the present disclosure relates to any one of the aforementioned methods, wherein the slit has a cruciate shape.

In certain embodiments, the present disclosure relates to any one of the aforementioned methods, wherein the mixture gels on the surface to form a biodegradable hydrogel.

In certain embodiments, the present disclosure relates to any one of the aforementioned methods, further comprising the step of agitating the vial to promote mixing of the mixture.

In certain embodiments, the present disclosure relates to any one of the aforementioned methods, further comprising the step of retracting and advancing the piston at least once to aid in the mixing of the first component and second component.

In certain embodiments, the present disclosure relates to any one of the aforementioned methods, further comprising the step of retracting and advancing the piston at least once to aid in the mixing of the first component and second component, thereby introducing air into the mixture of the first and second components (i.e., frothing the mixture).

In certain embodiments, the present disclosure relates to any one of the aforementioned methods, wherein the first component is a liquid. In certain embodiments, the present disclosure relates to any one of the aforementioned methods, wherein the liquid is a buffer. In certain embodiments, the present disclosure relates to any one of the aforementioned methods, wherein the liquid is a buffer solution comprising a cross-linking agent.

In certain embodiments, the present disclosure relates to any one of the aforementioned methods, wherein the separator is a hydrophobic barrier. In certain embodiments, the present invention relates to any one of the aforementioned methods, wherein the hydrophobic barrier is a hydrophobic filter.

In certain embodiments, the present disclosure relates to any one of the aforementioned methods, wherein instead of a separator, such as a hydrophobic barrier, the applicator comprises a floating plunger and a fluid bypass in the syringe barrel; wherein the floating plunger divides the syringe barrel into top and bottom compartments. The term "fluid bypass" as used herein refers to a structural aspect of, for example, a syringe barrel, that allows fluid to flow from one compartment to another once a plunger head, or the like, is distally advanced. See, for example, US Patent No. 4,735,616.

In certain embodiments, the present disclosure relates to any one of the aforementioned methods, wherein the separator is a two-way valve; and the valve is opened to allow for fluid communication between the top and bottom compartments before the piston is advanced.

In certain embodiments, the present disclosure relates to any one of the aforementioned methods, wherein the separator is a pierceable barrier; and the piston comprises a piercer which can pierce the separator, thereby allowing for fluid communication between the top and bottom compartments.

In certain embodiments, the present disclosure relates to any one of the aforementioned methods, wherein the separator is breakable when the syringe barrel is squeezed, thereby allowing for fluid communication between the top and bottom compartments; and the method further comprises the step of breaking the separator thereby placing the top and bottom compartments in fluid communication before advancing the piston.

In certain embodiments, the present disclosure relates to any one of the aforementioned methods, wherein the separator comprises one or more polymers selected from the group consisting of polyacrylics, silicones, polyolefins, polystyrenes, polyesters, polyethers, polyurethanes, polycarbonates, polyamines, or co-polymers thereof. In certain embodiments, the present invention relates to any one of the aforementioned methods, wherein the separator comprises a metal-containing laminate.

In certain embodiments, the present disclosure relates to any one of the aforementioned methods, wherein the applicator further comprises a cross-linking agent in the bottom compartment. In certain embodiments, the present disclosure relates to any one of the aforementioned methods, wherein the cross-linking agent is a polyalkyleneimine. In certain embodiments, the present disclosure relates to any one of the aforementioned methods, wherein the cross-linking agent is PEI.

In certain embodiments, the present disclosure relates to any one of the aforementioned methods, wherein the applicator further comprises a cross-linking agent in the bottom compartment; and a dissolution aid in the bottom compartment; wherein the cross-linking agent is adsorbed on the dissolution aid. In certain embodiments, the present disclosure relates to any one of the aforementioned methods, wherein the dissolution aid is a porous solid. In certain embodiments, the present disclosure relates to any one of the aforementioned methods, wherein the dissolution aid is a filter. In certain embodiments, the present disclosure relates to any one of the aforementioned methods, wherein the dissolution aid is affixed to the sidewall of the syringe barrel. In certain embodiments, the present disclosure relates to any one of the aforementioned methods, wherein the syringe barrel has a retention ring molded into the sidewall; and the retention ring immobilizes the dissolution aid.

In certain embodiments, the present disclosure relates to any one of the aforementioned methods, wherein the applicator further comprises a filter in the bottom compartment. In certain embodiments, the present disclosure relates to any one of the aforementioned methods, wherein the filter is affixed to the sidewall of the syringe barrel. In certain embodiments, the present disclosure relates to any one of the aforementioned methods, wherein the syringe barrel has a retention ring molded into the sidewall thereby immobilizing the filter. In certain embodiments, the present disclosure relates to any one of the aforementioned methods, wherein the filter knocks down air bubbles.

In certain embodiments, the present disclosure relates to any one of the aforementioned methods, wherein the sidewall of the syringe has a step in the bottom compartment (i.e., the syringe barrel has a stepped bore), so that the diameter at the distal end is smaller than the diameter at the proximal end.

In certain embodiments, the present disclosure relates to any one of the aforementioned methods, further comprising a retention ring which has a diameter approximately equal to the larger diameter of the stepped bore.

In certain embodiments, the present disclosure relates to any one of the aforementioned methods, further comprising a retention ring which has a diameter at its proximal end which is approximately equal to the larger diameter of the stepped bore, and a diameter at its distal end which is approximately equal to the smaller diameter of the stepped bore. In other words, the retention ring can fit over the step in the stepped bore.

In certain embodiments, the present disclosure relates to any one of the aforementioned methods, further comprising a dissolution aid or filter; wherein the dissolution aid or filter is held in place at the distal end of the barrel by the retention ring.

In certain embodiments, the present disclosure relates to any one of the aforementioned methods, wherein the retention ring is sonically welded in place.

In certain embodiments, the present disclosure relates to any one of the aforementioned methods, further comprising a sleeve insert in the distal end of the syringe barrel, thereby creating a stepped bore.

In certain embodiments, the present disclosure relates to any one of the aforementioned methods, further comprising a sleeve insert in the distal end of the syringe barrel, thereby creating a stepped bore; wherein the sleeve insert is ultrasonically welded in place.

In certain embodiments, the present disclosure relates to any one of the aforementioned methods, further comprising a sleeve insert in the distal end of the syringe barrel, thereby creating a stepped bore; and further comprising a dissolution aid or filter within the interior aspect of the sleeve insert.

In certain embodiments, the present disclosure relates to any one of the aforementioned methods, wherein the valve is a three-way valve, which in a first position connects the first and second fluid paths, in a second position connects the first and third fluid paths, and in a third position connects the second and third fluid paths.

In certain embodiments, the present disclosure relates to any one of the aforementioned methods, wherein attachment of the vial to the vial housing portion turns the valve so that the vial is in fluid communication with the bottom compartment. In certain embodiments, the present invention relates to any one of the aforementioned applicators, In certain embodiments, the present invention relates to any one of the aforementioned applicators, wherein the removal of the vial and the vial housing portion from the adapter turns the valve so that the fitting is in fluid communication with the bottom compartment.

In certain embodiments, the present disclosure relates to any one of the aforementioned methods, wherein the second component is a solid. In certain embodiments, the present disclosure relates to any one of the aforementioned methods, wherein the solid is an activated PEG. In certain embodiments, the present disclosure relates to any one of the aforementioned methods, wherein the activated PEG is a star, dendritic, or branched polymer with between three and less than twenty activating groups. In certain embodiments, the present disclosure relates to any one of the aforementioned methods, wherein the solid is 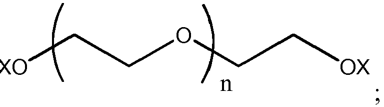 wherein n is 10-200 inclusive; and X is -CH₂C(=O)O(*N*-succinimidyl), -(CH₂)₂C(=O)O(*N*-succinimidyl), -(CH₂)₃ C(=O)O(*N*-succinimidyl),-(CH₂)₄C(=O)O(*N*-succinimidyl), -(CH₂)₅C(=O)O(*N*-succinimidyl), -(CH₂)₆C(=O)O(*N*-succinimidyl), -(CH₂)₇C(=O)O(*N*-succinimidyl), -(CH₂)₈C(=O)O(*N*-succinimidyl), -(CH₂)₉C(=O)O(*N*-succinimidyl), -C(=O)CH₂C(=O)O(*N*-succinimidyl), -C(=O)(CH₂)₂C(=O)O(*N*-succinimidyl),-C(=O)(CH₂)₃C(=O)O(*N*-succinimidyl), -C(=O)(CH₂)₄C(=O)O(*N*-succinimidyl), -C(=O)(CH₂)₅C(=O)O(*N*-succinimidyl), -C(=O)(CH₂)₆C(=O)O(*N*-succinimidyl), -C(=O)(CH₂)₇C(=O)O(*N*-succinimidyl), -C(=O)(CH₂)₈C(=O)O(*N*-succinimidyl), or -C(=O)(CH₂)₉C(=O)O(*N*-succinimidyl). In certain embodiments, the present disclosure relates to any one of the aforementioned methods, wherein the solid is 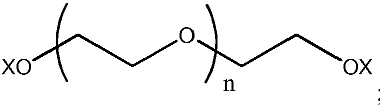 wherein n is 80-120 inclusive; and X is -(CH₂)₃C(=O)O(*N*-succinimidyl), -C(=O)(CH₂)₃C(=O)O(*N*-succinimidyl), or -C(=O)(CH₂)₈C(=O)O(*N*-succinimidyl).

In certain embodiments, the present disclosure relates to any one of the aforementioned methods, wherein the tip is a brush tip, a spray tip, a gas-assisted spray tip, a cannula, a needle, a flexible tube, a bent cannula, a shape-conforming tube, or a combination thereof. In certain embodiments, the present disclosure relates to any one of the aforementioned methods, wherein the tip is a blunt cannula. In certain embodiments, the present disclosure relates to any one of the aforementioned methods, wherein the fitting is a luer-lock adapter.

In certain embodiments, the present disclosure relates to any one of the aforementioned methods, wherein the length of the syringe barrel is between about 1.27 cm (0.5 inches) to about 22.86 cm (9 inches); or about 3.81 cm (1.5 inches) to about 10.16 cm (4 inches); or about 5.08 cm (2 inches) to about 7.62 cm (3 inches).

In certain embodiments, the present disclosure relates to any one of the aforementioned methods, wherein the diameter of the syringe barrel is between about 0.508 cm (0.2 inches) to about 5.08 cm (2 inches); or about 0.762 cm (0.3 inches) to about 1.905 cm (0.75 inches); or about 1.016 cm (0.4 inches) to about 1.524 cm (0.6 inches).

In certain embodiments, the present disclosure relates to any one of the aforementioned methods, wherein the applicator has a sterility assurance level of between about 10⁻³ to about 10⁻⁶.

### Sterilization Procedures

A variety of procedures can be used to sterilize the applicators and/or the chemical composition contained therein. Sterilization may be accomplished by, for example, chemical, physical, or irradiation techniques. Examples of chemical methods include exposure to ethylene oxide or hydrogen peroxide vapor. Examples of physical methods include sterilization by heat (dry or moist), retort canning, and filtration. The British Pharmacopoeia recommends heating at a minimum of 160 °C for not less than 2 hours, a minimum of 170 °C for not less than 1 hour and a minimum of 180 °C for not less than 30 minutes for effective sterilization. For examples of heat sterilization, see U.S. Patent 6,136,326. Passing the chemical composition through a membrane can be used to sterilize a composition. For example, the composition is filtered through a small pore filter such as a 0.22 micron filter which comprises material inert to the composition being filtered. In certain instances, the filtration is conducted in a Class 100,000 or better clean room. Examples of irradiation methods include gamma irradiation, electron beam irradiation, microwave irradiation, and irradiation using visible light. One preferred method is electron beam irradiation, as described in U.S. Patents 6,743,858; 6,248,800; and 6,143,805

There are several sources for electron beam irradiation. The two main groups of electron beam accelerators are: (1) a Dynamitron, which uses an insulated core transformer, and (2) radio frequency (RF) linear accelerators (linacs). The Dynamitron is a particle accelerator (4.5 MeV) designed to impart energy to electrons. The high energy electrons are generated and accelerated by the electrostatic fields of the accelerator electrodes arranged within the length of the glass-insulated beam tube (acceleration tube). These electrons, traveling through an extension of the evacuation beam tube and beam transport (drift pipe) are subjected to a magnet deflection system in order to produce a "canned" beam, prior to leaving the vacuum enclosure through a beam window. The dose can be adjusted with the control of the percent scan, the beam current, and the conveyor speed. In certain instances, the electron-beam radiation employed may be maintained at an initial fluence of at least about 2 µCurie/cm², at least about 5 µCurie/cm², at least about 8 µCuric/cm², or at least about 10 µCuric/cm². In certain instances, the electron-beam radiation employed has an initial fluence of from about 2 to about 25 µCurie/cm², In certain instances, the electron-beam dosage is from about 5 to 50 kGray (kGy), or from about 15 to about 20 kGy with the specific dosage being selected relative to the density of material being subjected to electron-beam radiation as well as the amount of bioburden estimated to be therein. Such factors are well within the skill of the art.

The applicators and/or composition to be sterilized may be in any type of at least partially electron beam permeable container such as glass or plastic. In embodiments of the present invention, the container may be sealed or have an opening. The penetration of electron beam irradiation is a function of the packaging. If there is not enough penetration from the side of a stationary electron beam, the container may be flipped or rotated to achieve adequate penetration. Alternatively, the electron beam source can be moved about a stationary package. In order to determine the dose distribution and dose penetration in product load, a dose map can be performed. This will identify the minimum and maximum dose zone within a product.

Procedures for sterilization using visible light are described in U.S. Patent 6,579,916. The visible light for sterilization can be generated using any conventional generator of sufficient power and breadth of wavelength to effect sterilization. Generators are commercially available under the trade name PureBright® in-line sterilization systems from PurePulse Technologies, Inc. 4241 Ponderosa Ave, San Diego, Calif. 92123, USA. The PureBright® in-line sterilization system employs visible light to sterilize clear liquids at an intensity approximately 90,000 times greater than surface sunlight. If the amount of UV light penetration is of concern, conventional UV absorbing materials can be used to filter out the UV light.

In a preferred embodiment, the composition in the applicator is sterilized to provide an applicator with a Sterility Assurance Level (SAL) of at least about 10⁻³. The Sterility Assurance Level measurement standard is described, for example, in ISO/CD 14937. In certain embodiments, the Sterility Assurance Level may be at least about 10⁻⁴, at least about 10⁻⁵, or at least about 10⁻⁶.

As discussed above, in certain embodiments of the present invention, one or more of the compositions, reagents, or components of a kit has been sterilized. The sterilization may be achieved using gamma radiation, e-beam radiation, dry heat sterilization, ethylene oxide sterilization, or a combination of any of them. The compositions, reagents, or components of the kits can be sterilized in an aqueous solution or neat.

In certain embodiments a compound present in an applicator (as described herein) has been sterilized by e-beam radiation between 2-40 kGy; or between 3-20 kGy; or between 5-12 kGy. In certain embodiments, said sterilization is carried out below 30 °C. In certain embodiments, said sterilization is carried out below 20 °C. In certain embodiments, said sterilization is carried out below 10 °C. In certain embodiments, said sterilization is carried out below 0 °C.

### Kits

In another aspect of the invention kits are provided comprising one or more applicators of the invention. A "kit," as used herein, typically defines a package or an assembly including one or more of the applicators of the invention, and/or other items, such as vials and tips, as described herein.

Herein is described a kit, comprising an applicator; a vial; and a tip. In certain embodiments, the present invention relates to any one of the aforementioned kits, wherein the applicator is affixed to a board. In certain embodiments, the present invention relates to any one of the aforementioned kits, wherein the applicator, vial and at least one tip are affixed to a board. In certain embodiments, the present invention relates to any one of the aforementioned kits, wherein the board is in a foil pouch. In certain embodiments, the present invention relates to any one of the aforementioned kits, further comprising an oxygen absorber affixed to the board.

Herein is described any one of the aforementioned kits, wherein the applicator comprises (1) a syringe barrel comprising a sidewall; a separator; a piston; a proximal end; and a distal end; wherein the piston seals the proximal end of the syringe barrel and is slideable within the syringe barrel; the separator divides the syringe barrel into a top compartment, bounded by the piston and the separator, and a bottom compartment, bounded by the distal end of the syringe barrel and the separator; and the distal end is configured to attach to an adapter; and (2) a first component in the top compartment.

Herein is described any one of the aforementioned kits, further comprising an adapter comprising a removable vial housing portion comprising a piercing structure; a syringe housing portion configured to attach to the distal end of a syringe barrel; a fitting configured to attach to a tip; a valve; a first fluid pathway connecting the syringe to the valve; a second fluid pathway connecting the vial housing portion to the valve; and a third fluid pathway connecting the fitting to the valve. In certain embodiments, the present invention relates to any one of the aforementioned kits, wherein the adapter is packaged attached to the syringe barrel.

In certain embodiments, the present invention relates to any one of the aforementioned kits, further comprising a cap over the fitting.

In certain embodiments, the present invention relates to any one of the aforementioned kits, further comprising a cap over the piercing structure. In certain embodiments, the present invention relates to any one of the aforementioned kits, wherein the piercing structure cap is affixed to the board so that when the applicator is removed from the board the cap remains affixed to the board and is removed from the piercing structure.

In certain embodiments, the present invention relates to any one of the aforementioned kits, wherein the first component is a liquid. In certain embodiments, the present invention relates to any one of the aforementioned kits, wherein the liquid is a buffer. In certain embodiments, the present invention relates to any one of the aforementioned kits, wherein the liquid is a buffer solution comprising a cross-linking agent.

In certain embodiments, the present invention relates to any one of the aforementioned kits, wherein the separator is a hydrophobic barrier. In certain embodiments, the present invention relates to any one of the aforementioned kits, wherein the hydrophobic barrier is a hydrophobic filter.

In certain embodiments, the present invention relates to any one of the aforementioned kits, wherein instead of a separator, such as a hydrophobic barrier, the applicator comprises a floating plunger and a fluid bypass in the syringe barrel; wherein the floating plunger divides the syringe barrel into top and bottom compartments. The term "fluid bypass" as used herein refers to a structural aspect of, for example, a syringe body, that allows fluid to flow from one compartment to another once a plunger head, or the like, is distally advanced. See, for example, US Patent No. 4,735,616.

In certain embodiments, the present invention relates to any one of the aforementioned kits, wherein the separator is a two-way valve.

In certain embodiments, the present invention relates to any one of the aforementioned kits, wherein the separator is a pierceable barrier; and the piston comprises a piercer which can pierce the separator, thereby allowing for fluid communication between the top and bottom compartments.

In certain embodiments, the present invention relates to any one of the aforementioned kits, wherein the separator is breakable when the syringe barrel is squeezed, thereby allowing for fluid communication between the top and bottom compartments.

In certain embodiments, the present invention relates to any one of the aforementioned kits, wherein the separator comprises one or more polymers selected from the group consisting of polyacrylics, silicones, polyolefins, polystyrenes, polyesters, polyethers, polyurethanes, polycarbonates, polyamines, or co-polymers thereof. In certain embodiments, the present invention relates to any one of the aforementioned kits, wherein the separator comprises a metal-containing laminate.

In certain embodiments, the present invention relates to any one of the aforementioned kits, further comprising a cross-linking agent in the bottom compartment. In certain embodiments, the present invention relates to any one of the aforementioned kits, wherein the cross-linking agent is a polyalkyleneimine. In certain embodiments, the present invention relates to any one of the aforementioned kits, wherein the cross-linking agent is PEI.

In certain embodiments, the present invention relates to any one of the aforementioned kits, further comprising a cross-linking agent in the bottom compartment; and a dissolution aid in the bottom compartment; wherein the cross-linking agent is adsorbed on the dissolution aid. In certain embodiments, the present invention relates to any one of the aforementioned kits, wherein the dissolution aid is a porous solid. In certain embodiments, the present invention relates to any one of the aforementioned kits, wherein the dissolution aid is a filter. In certain embodiments, the present invention relates to any one of the aforementioned kits, wherein the dissolution aid is affixed to the sidewall of the syringe barrel. In certain embodiments, the present invention relates to any one of the aforementioned kits, wherein the syringe barrel has a retention ring molded into the sidewall; and the retention ring immobilizes the dissolution aid.

In certain embodiments, the present invention relates to any one of the aforementioned kits, further comprising a filter in the bottom compartment. In certain embodiments, the present invention relates to any one of the aforementioned kits, wherein the filter is affixed to the sidewall of the syringe barrel. In certain embodiments, the present invention relates to any one of the aforementioned kits, wherein the syringe barrel has a retention ring molded into the sidewall thereby immobilizing the filter. In certain embodiments, the present invention relates to any one of the aforementioned kits, wherein the filter knocks down air bubbles.

In certain embodiments, the present invention relates to any one of the aforementioned kits, wherein the sidewall of the syringe has a step in the bottom compartment (i.e., the syringe barrel has a stepped bore), so that the diameter at the distal end is smaller than the diameter at the proximal end.

In certain embodiments, the present invention relates to any one of the aforementioned kits, further comprising a retention ring which has a diameter approximately equal to the larger diameter of the stepped bore.

In certain embodiments, the present invention relates to any one of the aforementioned kits, further comprising a retention ring which has a diameter at its proximal end which is approximately equal to the larger diameter of the stepped bore, and a diameter at its distal end which is approximately equal to the smaller diameter of the stepped bore. In other words, the retention ring can fit over the step in the stepped bore.

In certain embodiments, the present invention relates to any one of the aforementioned kits, further comprising a dissolution aid or filter; wherein the dissolution aid or filter is held in place at the distal end of the barrel by the retention ring.

In certain embodiments, the present invention relates to any one of the aforementioned kits, wherein the retention ring is sonically welded in place.

In certain embodiments, the present invention relates to any one of the aforementioned kits, further comprising a sleeve insert in the distal end of the syringe barrel, thereby creating a stepped bore.

In certain embodiments, the present invention relates to any one of the aforementioned kits, further comprising a sleeve insert in the distal end of the syringe barrel, thereby creating a stepped bore; wherein the sleeve insert is ultrasonically welded in place.

In certain embodiments, the present invention relates to any one of the aforementioned kits, further comprising a sleeve insert in the distal end of the syringe barrel, thereby creating a stepped bore; and further comprising a dissolution aid or filter within the interior aspect of the sleeve insert.

In certain embodiments, the present invention relates to any one of the aforementioned kits, wherein the valve is a three-way valve, which in a first position connects the first and second fluid paths, in a second position connects the first and third fluid paths, and in a third position connects the second and third fluid paths.

In certain embodiments, the present invention relates to any one of the aforementioned kits, further comprising a vial reversibly attached to the vial housing portion; wherein the vial comprises a second component; the vial is capped by a septum; and the septum is pierced by the piercing structure when the vial is reversibly attached to the vial housing portion, thereby placing the vial in fluid communication with the adapter through the second fluid pathway. In certain embodiments, the present invention relates to any one of the aforementioned kits, wherein attachment of the vial to the vial housing portion turns the valve so that the vial is in fluid communication with the bottom compartment. In certain embodiments, the present invention relates to any one of the aforementioned kits, wherein the removal of the vial and the vial housing portion from the adapter turns the valve so that the fitting is in fluid communication with the bottom compartment.

In certain embodiments, the present invention relates to any one of the aforementioned kits, wherein the second component is a solid. In certain embodiments, the present invention relates to any one of the aforementioned kits, wherein the solid is an activated PEG. In certain embodiments, the present invention relates to any one of the aforementioned kits, wherein the activated PEG is a star, dendritic, or branched polymer with between three and less than twenty activating groups. In certain embodiments, the present invention relates to any one of the aforementioned kits, wherein the solid is 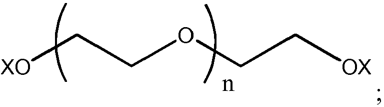 wherein n is 10-200 inclusive; and X is -CH₂C(=O)O(*N*-succinimidyl), -(CH₂)₂C(=O)O(*N*-succinimidyl), -(CH₂)₃C(=O)O(*N*-succinimidyl), -(CH₂)₄C(=O)O(*N*-succinimidyl), -(CH₂)₅C(=O)O(*N*-succinimidyl), -(CH₂)₆C(=O)O(*N*-succinimidyl), -(CH₂)₇C(=O)O(*N*-succinimidyl), -(CH₂)₈C(=O)O(*N*-succinimidy1), -(CH₂)₉C(=O)O(*N*-succinimidyl), -C(=O)CH₂C(=O)O(*N*-succinimidyl), -C(=O)(CH₂)₂C(=O)O(*N*-succinimidyl), -C(=O)(CH₂)₅C(=O)O(*N*-succinimidyl), -C(=O)(CH₂)₆C(=O)O(*N*-succinimidyl), -C(=O)(CH₂)₇C(=O)O(*N*-succinimidyl), -C(=O)(CH₂)₈C(=O)O(*N*-succinimidyl), or -C(=O)(CH₂)₉C(=O)O(*N*-succinimidyl). In certain embodiments, the present invention relates to any one of the aforementioned kits, wherein the solid is 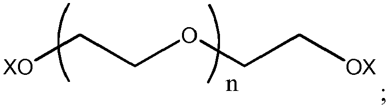 wherein n is 80-120 inclusive; and X is -(CH₂)₃C(=O)O(*N*-succinimidyl), -C(=O)(CH₂)₃C(=O)O(*N*-succinimidyl), or -C(=O)(CH₂)₈C(=O)O(*N*-succinimidyl).

In certain embodiments, the present invention relates to any one of the aforementioned kits, further comprising a tip attached to the fitting. In certain embodiments, the present invention relates to any one of the aforementioned kits, wherein the tip is a brush tip, a spray tip, a gas-assisted spray tip, a cannula, a needle, a flexible tube, a bent cannula, a shape-conforming tube, or a combination thereof. In certain embodiments, the present invention relates to any one of the aforementioned kits, wherein the tip is a blunt cannula. In certain embodiments, the present invention relates to any one of the aforementioned kits, wherein the fitting is a luer-lock adapter.

In certain embodiments, the present invention relates to any one of the aforementioned kits, wherein the length of the syringe barrel is between about 1.27 cm (0.5 inches) to about 22.86 cm (9 inches); or about 3.81 cm (1.5 inches) to about 10.16 cm (4 inches); or about 5.08 cm (2 inches) to about 7.62 cm (3 inches).

In certain embodiments, the present invention relates to any one of the aforementioned kits, wherein the diameter of the syringe barrel is between about 0.508 cm (0.2 inches) to about 5.08 cm (2 inches); or about 0.762 cm (0.3 inches) to about 1.905 cm (0.75 inches); or about 1.016 cm (0.4 inches) to about 1.524 cm (0.6 inches).

In certain embodiments, the present invention relates to any one of the aforementioned kits, wherein two of any of the aforementioned applicators are provided; wherein the tip has a first inlet, a second inlet and an outlet. Such a kit could be used to assemble an applicator such as the one shown in Figure 3.

Herein is described any one of the aforementioned kits, wherein the applicator comprises (1) a syringe barrel comprising a sidewall; a separator; a piston; a proximal end; and a distal end; wherein the piston seals the proximal end of the syringe barrel and is slideable within the syringe barrel; the separator divides the syringe barrel into a top compartment, bounded by the piston and the separator, and a bottom compartment, bounded by the distal end of the syringe barrel and the separator; and the distal end comprises a fitting configured to attach to a tip; (2) a vial capped by a septum; wherein the septum has a slit through which a tip can pass; (3) a first component in the top compartment; and (4) a second component in the vial.

In certain embodiments, the present invention relates to any one of the aforementioned kits, wherein the slit has a cruciate shape.

In certain embodiments, the present invention relates to any one of the aforementioned kits, wherein the first component is a liquid. In certain embodiments, the present invention relates to any one of the aforementioned kits, wherein the liquid is a buffer. In certain embodiments, the present invention relates to any one of the aforementioned kits, wherein the liquid is a buffer solution comprising a cross-linking agent.

In certain embodiments, the present invention relates to any one of the aforementioned kits, wherein the separator is a hydrophobic barrier. In certain embodiments, the present invention relates to any one of the aforementioned kits, wherein the hydrophobic barrier is a hydrophobic filter.

In certain embodiments, the present invention relates to any one of the aforementioned kits, wherein instead of a separator, such as a hydrophobic barrier, the applicator comprises a floating plunger and a fluid bypass in the syringe barrel; wherein the floating plunger divides the syringe barrel into top and bottom compartments. The term "fluid bypass" as used herein refers to a structural aspect of, for example, a syringe body, that allows fluid to flow from one compartment to another once a plunger head, or the like, is distally advanced. See, for example, US Patent No. 4,735,616.

In certain embodiments, the present invention relates to any one of the aforementioned kits, wherein the separator is a two-way valve.

In certain embodiments, the present invention relates to any one of the aforementioned kits, wherein the separator is a pierceable barrier; and the piston comprises a piercer which can pierce the separator, thereby allowing for fluid communication between the top and bottom compartments.

In certain embodiments, the present invention relates to any one of the aforementioned kits, wherein the separator is breakable when the syringe barrel is squeezed, thereby allowing for fluid communication between the top and bottom compartments.

In certain embodiments, the present invention relates to any one of the aforementioned kits, wherein the separator comprises one or more polymers selected from the group consisting of polyacrylics, silicones, polyolefins, polystyrenes, polyesters, polyethers, polyurethanes, polycarbonates, polyamines, or co-polymers thereof. In certain embodiments, the present invention relates to any one of the aforementioned kits, wherein the separator comprises a metal-containing laminate.

In certain embodiments, the present invention relates to any one of the aforementioned kits, further comprising a cross-linking agent in the bottom compartment. In certain embodiments, the present invention relates to any one of the aforementioned kits, wherein the cross-linking agent is a polyalkyleneimine. In certain embodiments, the present invention relates to any one of the aforementioned kits, wherein the cross-linking agent is PEI.

In certain embodiments, the present invention relates to any one of the aforementioned kits, further comprising a cross-linking agent in the bottom compartment; and a dissolution aid in the bottom compartment; wherein the cross-linking agent is adsorbed on the dissolution aid. In certain embodiments, the present invention relates to any one of the aforementioned kits, wherein the dissolution aid is a porous solid. In certain embodiments, the present invention relates to any one of the aforementioned kits, wherein the dissolution aid is a filter. In certain embodiments, the present invention relates to any one of the aforementioned kits, wherein the dissolution aid is affixed to the sidewall of the syringe barrel. In certain embodiments, the present invention relates to any one of the aforementioned kits, wherein the syringe barrel has a retention ring molded into the sidewall; and the retention ring immobilizes the dissolution aid.

In certain embodiments, the present invention relates to any one of the aforementioned kits, further comprising a filter in the bottom compartment. In certain embodiments, the present invention relates to any one of the aforementioned kits, wherein the filter is affixed to the sidewall of the syringe barrel. In certain embodiments, the present invention relates to any one of the aforementioned kits, wherein the syringe barrel has a retention ring molded into the sidewall thereby immobilizing the filter. In certain embodiments, the present invention relates to any one of the aforementioned kits, wherein the filter knocks down air bubbles.

In certain embodiments, the present invention relates to any one of the aforementioned kits, wherein the sidewall of the syringe has a step in the bottom compartment (i.e., the syringe barrel has a stepped bore), so that the diameter at the distal end is smaller than the diameter at the proximal end.

In certain embodiments, the present invention relates to any one of the aforementioned kits, further comprising a retention ring which has a diameter approximately equal to the larger diameter of the stepped bore.

In certain embodiments, the present invention relates to any one of the aforementioned kits, further comprising a retention ring which has a diameter at its proximal end which is approximately equal to the larger diameter of the stepped bore, and a diameter at its distal end which is approximately equal to the smaller diameter of the stepped bore. In other words, the retention ring can fit over the step in the stepped bore.

In certain embodiments, the present invention relates to any one of the aforementioned kits, further comprising a dissolution aid or filter; wherein the dissolution aid or filter is held in place at the distal end of the barrel by the retention ring.

In certain embodiments, the present invention relates to any one of the aforementioned kits, wherein the retention ring is sonically welded in place.

In certain embodiments, the present invention relates to any one of the aforementioned kits, further comprising a sleeve insert in the distal end of the syringe barrel, thereby creating a stepped bore.

In certain embodiments, the present invention relates to any one of the aforementioned kits, further comprising a sleeve insert in the distal end of the syringe barrel, thereby creating a stepped bore; wherein the sleeve insert is ultrasonically welded in place.

In certain embodiments, the present invention relates to any one of the aforementioned kits, further comprising a sleeve insert in the distal end of the syringe barrel, thereby creating a stepped bore; and further comprising a dissolution aid or filter within the interior aspect of the sleeve insert.

In certain embodiments, the present invention relates to any one of the aforementioned kits, further comprising a tip attached to the fitting. In certain embodiments, the present invention relates to any one of the aforementioned kits, wherein the tip is a brush tip, a spray tip, a gas-assisted spray tip, a cannula, a needle, a flexible tube, a bent cannula, a shape-conforming tube, or a combination thereof. In certain embodiments, the present invention relates to any one of the aforementioned kits, wherein the tip is a blunt cannula. In certain embodiments, the present invention relates to any one of the aforementioned kits, wherein the fitting is a luer-lock adapter.

In certain embodiments, the present invention relates to any one of the aforementioned kits, wherein the length of the syringe barrel is between about 1.27 cm (0.5 inches) to about 22.86 cm (9 inches); or about 3.81 cm (1.5 inches) to about 10.16 cm (4 inches); or about 5.08 cm (2 inches) to about 7.62 cm (3 inches).

In certain embodiments, the present invention relates to any one of the aforementioned kits, wherein the diameter of the syringe barrel is between about 0.508 cm (0.2 inches) to about 5.08 cm (2 inches); or about 0.762 cm (0.3 inches) to about 1.905 cm (0.75 inches); or about 1.016 cm (0.4 inches) to about 1.524 cm (0.6 inches).

In certain embodiments, the present invention relates to any one of the aforementioned kits, wherein the second component is a solid. In certain embodiments, the present invention relates to any one of the aforementioned kits, wherein the solid is an activated PEG. In certain embodiments, the present invention relates to any one of the aforementioned kits, wherein the activated PEG is a star, dendritic, or branched polymer with between three and less than twenty activating groups. In certain embodiments, the present invention relates to any one of the aforementioned kits, wherein the solid is 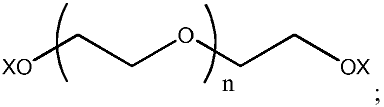 wherein n is 10-200 inclusive; and X is -CH₂C(=O)O(*N*-succinimidyl),-(CH₂)₂C(=O)O(*N*-succinimidyl),-(CH₂)₃C(=O)O(*N*-succinimidyl), -(CH₂)₄C(=O)O(*N*-succinimidyl), -(CH₂)₅C(=O)O(*N*-succinimidyl), -(CH₂)₆C(=O)O(*N*-succinimidyl), -(CH₂)₇C(=O)O(*N*-succinimidyl), -(CH₂)₈C(=O)O(*N*-succinimidyl), -(CH₂)₉C(=O)O(*N*-succinimidyl), -C(=O)CH₂C(=O)O(*N*-succinimidyl), -C(=O)(CH₂)₂C(=O)O(*N*-succinimidyl), -C(=O)(CH₂)₃C(=O)O(*N*-succinimidyl), -C(=O)(CH₂)₄C(=O)O(*N*-succinimidyl), -C(=O)(CH₂)₅C(=O)O(*N*-succinimidyl), -C(=O)(CH₂)₆C(=O)O(*N*-succinimidyl), -C(=O)(CH₂)₇C(=O)O(*N*-succinimidyl), -C(=O)(CH₂)₈C(=O)O(*N*-succinimidyl), or -C(=O)(CH₂)₉C(=O)O(*N*-succinimidyl). In certain embodiments, the present invention relates to any one of the aforementioned kits, wherein the solid is 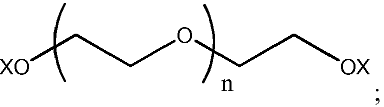 wherein n is 80-120 inclusive; and X is -(CH₂)₃C(=O)O(*N*-succinimidyl), -C(=O)(CH₂)₃C(=O)O(*N*-succinimidyl), or -C(=O)(CH₂)₈C(=O)O(*N*-succinimidyl).

In certain embodiments, the present invention relates to any one of the aforementioned kits, wherein the tip is a blunt cannula.

In certain embodiments, the present invention relates to any one of the aforementioned kits, wherein the kit has a sterility assurance level of between about 10⁻³ to about 10⁻⁶.

In addition, in certain embodiments, such kits may include associated devices used to perform medical procedures. Each of the compositions of the kit may be provided in liquid form (e.g., in solution), or in solid form (e.g., a dried powder). In certain cases, some of the compositions may be constitutable or otherwise processable (e.g., to an active form), for example, by the addition of a suitable solvent or other species, which may or may not be provided with the kit. Examples of other compositions or components associated with the invention include, but are not limited to, solvents, surfactants, diluents, salts, buffers, emulsifiers, chelating agents, fillers, antioxidants, binding agents, bulking agents, preservatives, drying agents, antimicrobials, needles, syringes, packaging materials, tubes, bottles, flasks, beakers, dishes, frits, filters, rings, clamps, wraps, patches, containers, and the like, for example, for using, modifying, assembling, storing, packaging, preparing, mixing, diluting, and/or preserving the compositions components for a particular use. In certain embodiments, different parts of the applicators may be packaged separately (e.g., in Mylar pouches).

A kit of the invention may include instructions in any form that are provided in connection with the applicators of the invention in such a manner that one of ordinary skill in the art would recognize that the instructions are to be associated with the compositions of the invention. For instance, the instructions may relate to the use, modification, mixing, diluting, preserving, assembly, storage, packaging, and/or preparation of the applicators and/or other compositions associated with the kit. In some cases, the instructions may also include instructions for the use of the applicators. The instructions may be provided in any form recognizable by a user as a suitable vehicle for containing such instructions; for example, written or published, verbal, audible (e.g., telephonic), digital, optical, visual (e.g., videotape, DVD, etc.) or electronic communications (including Internet or web-based communications), provided in any manner.

### Definitions

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03.

A "trocar" is a hollow cylinder with a sharply pointed end, often three-sided, that is used to introduce cannulas and other similar implements into blood vessels or body cavities. Trocars are also used as ports in laparoscopic surgery. A trocar is often passed inside a cannula, and functions as a portal for the subsequent placement of other devices, such as a chest drain or intravenous cannula. In certain embodiments described herein, the nozzle (dispensing tip) of the apparatus is designed to pass through a trocar port or equivalent on an endoscope or laparoscope.

The term "brush" or "brush cannula" as used herein is known to those skilled in the art. The name represents the function of the brush: It is constructed to enable liquid to flow through the bristles for an application. The brushes can be attached to a wide variety of media that dispense liquid, and can be made out of many types of bristle material and configurations. In certain embodiments herein the brush cannula is connected to an applicator body. Brush cannulas are also known as flow-through or flow-thru brushes; the terms are used interchangeably herein.

The term "activated PEG" as used herein is known to those skilled in the art and refers to poly(ethylene) glycols (both linear and branched) which have either at least one end activated for conjugation with other molecules. Shown below are chemical structures for polyethylene glycol (PEG), mono-methylated polyethylene glycol (mPEG), an activated mPEG and a bis-activated PEG. 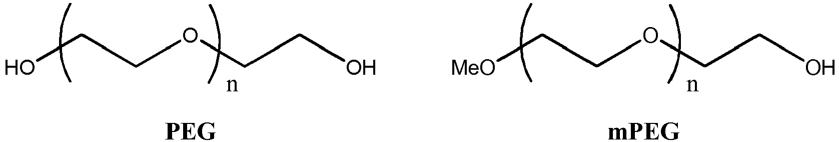 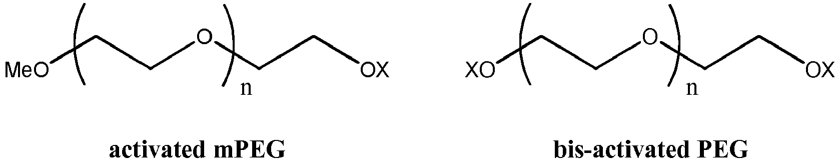 In the structures provided above n is a positive integer. In a batch of activated PEG different individual molecules will have a different values of n (i.e., the mixture is polydisperse); these mixtures are often characterized by an average molecular weight, which can be converted into an average value for n. In certain embodiments herein, the average n is between about 10 and about 200. In other embodiments the average n is between about 80 and about 120. In yet other embodiments, the average n is about 100. In the structures provided above, X can comprise a variety of chemical moieties such as, for example, a *N*-succinimide, a *N*-maleimide, a nitro, an aldehyde, an amine, a thiol, a ketal, an acetal, or a carbonate. In certain embodiments, X is selected from the group consisting of -CH₂C(=O)O(*N*-succinimidyl), -(CH₂)₂C(=O)O(*N*-succinimidyl), -(CH₂)₃C(=O)O(*N*-succinimidyl) ["PEG-SPA"], -(CH₂)₄C(=O)O(*N*-succinimidyl), -(CH₂)₅C(=O)O(*N*-succinimidyl), -(CH₂)₆C(=O)O(*N*-succinimidyl), -(CH₂)₇C(=O)O(*N*-succinimidyl), -(CH₂)₈C(=O)O(*N*-succinimidyl), -(CH₂)₉C(=O)O(*N*-succinimidyl), -C(=O)CH₂C(=O)O(*N*-succinimidyl), -C(=O)(CH₂)₂C(=O)O(*N*-succinimidyl), -C(=O)(CH₂)₃C(=O)O(*N*-succinimidyl)["PEG-SG"], -C(=O)(CH₂)₄C(=O)O(*N*-succinimidyl) ["PEG-adipate"], -C(=O)(CH₂)₅C(=O)O(*N*-succinimidyl), -C(=O)(CH₂)₆C(=O)O(*N*-succinimidyl), -C(=O)(CH₂)₇C(=O)O(*N*-succinimidyl), -C(=O)(CH₂)₈C(=O)O(*N*-succinimidyl)["PEG-sebacate"], -C(=O)(CH₂)₉C(=O)O(*N*-succinimidyl), -C(=O)(*p*-nitrophenyl), -CH₂CH₂C(=O)H, -CH₂CH₂CH₂NH₂, -CH₂CH₂CH(OCH₂CH₃)₂, -CH₂CH₂SH, -CH₂CH₂CH₂N(H)C(=O)CH₂CH₂(*N*-maleimidyl), and -O(C=O)O(*p*-nitrophenyl).

The term "PEG(NHS)₂" refers to a linear polyethylene glycol having -C(=O)O((*N*-succinimidyl) at both ends of the polymer chain. PEG(NHS)₂ can be prepared in variety of ways, such as by using either of the following methods. In method 1, a polyethylene glycol is subjected to oxidative conditions in order to oxidize the two termini to the corresponding carboxylic acids [HO₂CCH₂O-PEG-OCH₂CO₂H], followed by transformation to the bis(NHS ester). In method 2, PEG(NHS)₂ is prepared by alkylation of the two termini of a polyethylene glycol with acrylonitrile to give NCCH₂CH₂O-PEG-OCH₂CH₂CN, followed by hydrolysis to the bis(acid) [HO₂CCH₂CH₂O-PEG-OCH₂CH₂CO₂H], and then transformation to the bis(NHS ester).

As used here, "PEG-SPA" refers to the following structure: 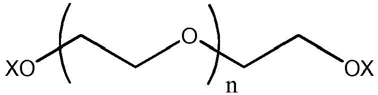 wherein X is -(CH₂)₃C(=O)O(*N*-succinimidyl); and n is an integer (e.g., from 10 to 200).

As used herein, "PEG-SG" refers to the following structure: 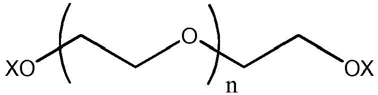 wherein X is -C(=O)(CH₂)₃C(=O)O(*N*-succinimidyl); and n is an integer (e.g., from 10 to 200).

As used herein, "PEG-adipate" refers to the following structure: 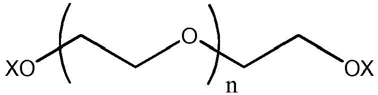 wherein X is -C(=O)(CH₂)₄C(=O)O(*N*-succinimidyl); and n is an integer (e.g., from 10 to 200).

As used herein, "PEG-sebacate" refers to the following structure: 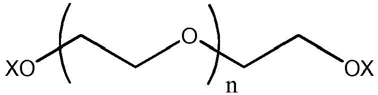 wherein X is -C(=O)(CH₂)₈C(=O)O(*N*-succinimidyl); and n is an integer (e.g., from 10 to 200).

As used herein, "plastic" refers to polyacrylics, silicones, polyolefins, polystyrenes, polyesters, polyethers, polyurethanes, polycarbonates, polyamines, or co-polymers thereof.

As used herein, "silicones" (polymerized siloxanes or polysiloxanes) are mixed inorganic-organic polymers with the chemical formula [R₂SiO]ₙ, where R may be an organic group such as methyl, ethyl, and phenyl. These materials consist of an inorganic silicon-oxygen backbone with organic side groups attached to the silicon atoms, which are four-coordinate. In some cases organic side groups can be used to link two or more of these backbones together. By varying the -Si-O-chain lengths, side groups, and cross-linking, silicones can be synthesized with a wide variety of properties and compositions.

As used herein, the term "patient" refers to any animal in need, including primates, in particular humans, and other mammals such as equines, cattle, swine and sheep; and poultry and pets in general.

As used herein, the term "reconstitution" means the mixing of more than one component into a formulation or formulation part which is at least meta-stable for some amount of time. It also includes dissolution (i.e., the process in which one substance is dissolved in another). In certain embodiments, the individual components may not be stable in the "reconstituted" state or may suffer from other difficulties such as tolerance to sterilization procedures which makes it necessary for the components to be separate during the bulk of the storage time of the device but allow for it to be "reconstituted" into a formulation or formulation part prior to application.

As used herein, "adsorption" is the adhesion of molecules of a gas, liquid or solid to a surface.

As used herein, a "piston" is a solid disk or cylinder that fits inside a hollow cylinder, and moves under pressure.

As used herein a "board" is a retainer organizing separate components within a kit. The "board" can be made from any relatively rigid material such as paper board, Solid Bleached Sulfate (SBS), or plastic. Furthermore a "board" can be a flat two dimensional die cut retainer or a three dimensional retainer such as a thermoformed blister or clamshell all of which are well known to those skilled in the art.

### EXEMPLIFICATION

The invention now being generally described, it will be more readily understood by reference to the following examples which are included merely for purposes of illustration of certain aspects and embodiments of the present disclosure, and are not intended to limit the invention.

### Example 1

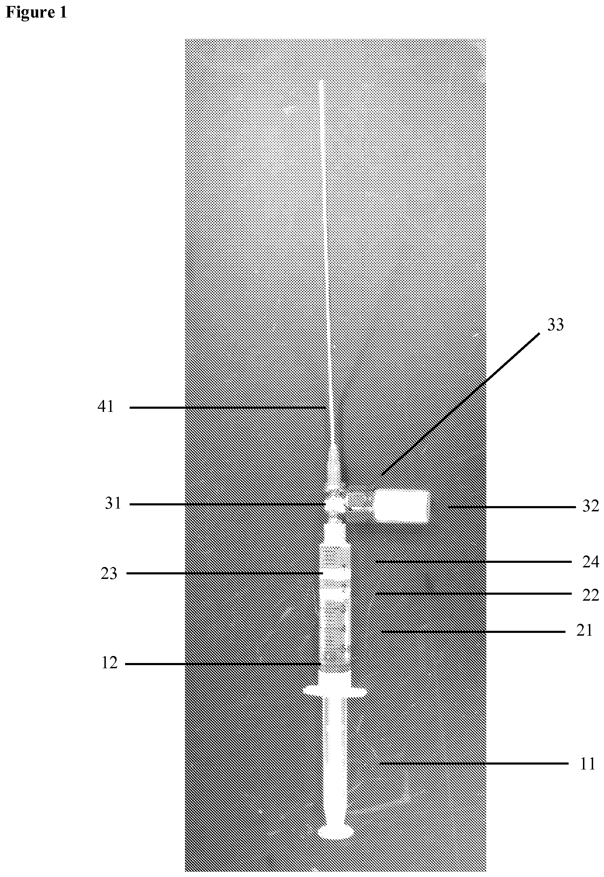

Figure 1 shows one embodiment of an applicator. The applicator as shown comprises a plunger (11) attached to a piston (12) which can be advanced through a syringe barrel. The syringe barrel is divided into an upper compartment (21) and a lower compartment (24) by a separator (22), which as shown is a hydrophobic barrier. Liquid can be placed in the upper compartment and can be pushed into the lower compartment when the piston advances towards the hydrophobic barrier. The lower compartment contains a dissolution aid (23) upon which a component of a formulation can be absorbed. The applicator further comprises an adapter (31) which connects the syringe barrel to a vial (32) via a vial housing (33). The adapter also can connect a tip (41).

Figure 3 depicts an applicator wherein the tip is bifurcated to allow two syringe barrels to be attached, via two adapters. The materials in the syringe barrels can be the same or different, as can the material in the vials.

### Example 2

The applicator of Figure 1 can be used for the preparation and delivery of a fully synthetic, absorbable hydrogel sealant. The sterile applicator is supplied in three parts, a syringe assembly containing polyethylenimine (PEI) and a buffer solution, a vial containing a polyethylene glycol (PEG) ester powder, and two angled applicator tips of varying length. Following attachment of the vial to the vial adapter on the syringe assembly and attachment of one of the angled applicator tips to the syringe assembly, the device is ready for use.

When the buffered PEI solution is mixed with the PEG powder inside the device, the PEG powder dissolves and the precursors begin to crosslink. Following complete reconstitution and removal of the vial adapter assembly, the mixed solution is delivered by depressing the syringe plunger, forcing the solution down the angled applicator and out of the tip. The delivered material cross-links to form a hydrogel sealant within approximately 30 seconds.

The resulting hydrogel sealant is absorbed over approximately 80 days, sufficient time to allow for healing.

### Example 3

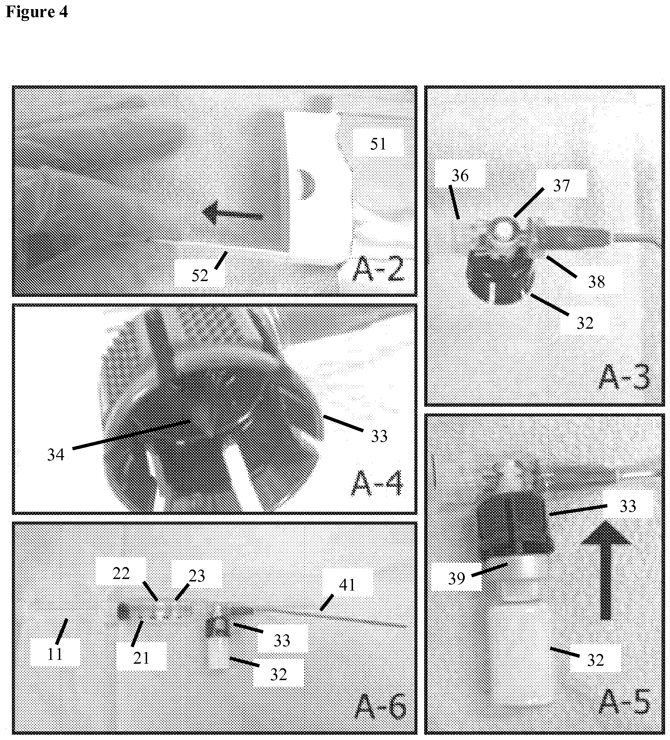

One method of assembling the applicator shown in Figure 1 is as follows. A foil pouch (51) containing the pieces of the applicator is opened, and a pull tab (52) is used to remove the retainer card upon which the applicator pieces are affixed (Figure 4, A-2). The syringe barrel connected to adapter (at 36) is then removed and one appropriately sized cannula (41) is selected and removed from the retainer card. The cannula is then attached to the syringe via the adapter (at 38) (Figure 4, A-3). A silicone cap is then removed from the piecing structure (34) of the vial housing assembly (Figure 4, A-4). A vial (32) is then removed from the foil pouch. The vial septum (39) is pressed against the piercing structure of the vial housing assembly until the vial is fully captured by the vial housing assembly (33) (Figure 4, A-5). The device is now ready for use (Figure 4, A-6). The assembled device may be held at this point for several hours without any deleterious effect.

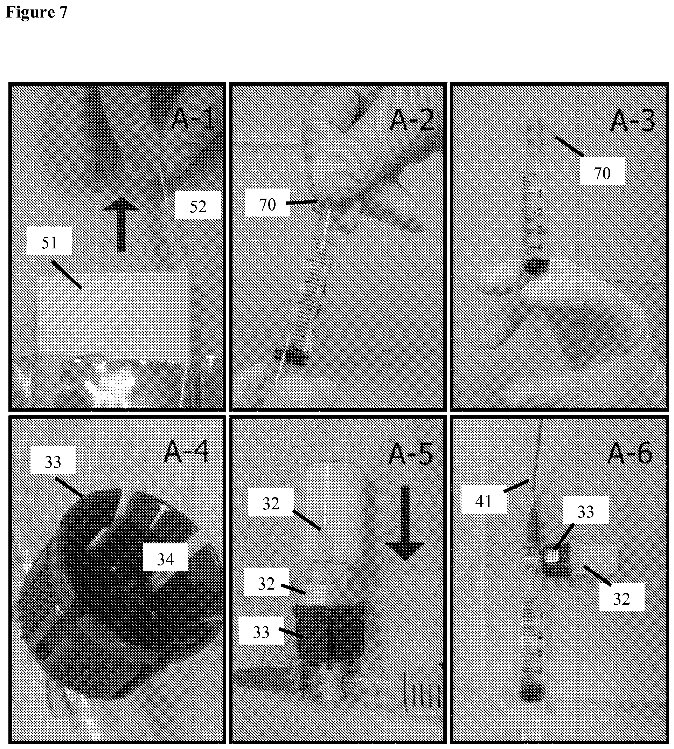

Another method of assembling the applicator shown in Figure 1 is as follows. A foil pouch (51) containing the pieces of the applicator is opened, and a pull tab (52) is used to remove the retainer card upon which the applicator pieces are affixed (Figure 7, A-1). The syringe barrel connected to a winged luer cap (70) is removed from the retainer card. After ensuring that the winged luer cap (70) is securely tightened onto the syringe assembly (Figure 7, A-2), the syringe is held upright and the plunger is depressed slowly to allow the buffer solution to pass through two filters: the separator and the dissolution aid (Figure 7, A-3). The plunger is then released to allow the solution to pass back through the filters. This is repeated at least three times to ensure that the contents are thoroughly dissolved. An adapter is then removed from the card and a silicone cap is removed from the piercing structure of the vial housing assembly (Figure 7, A-4). While holding the syringe upright, the winged luer cap is carefully removed and the adapter is attached in its place. An appropriately sized cannula is then removed from the retainer card and attached to the syringe via the adapter. A vial (32) is then removed from the foil pouch. The vial septum (39) is pressed against the piecing structure of the vial housing assembly until the vial is fully captured by the vial housing assembly (33) (Figure 7, A-5). The device is now ready for use (Figure 7, A-6). The assembled device may be held at this point for several hours without any deleterious effect.

### Example 4

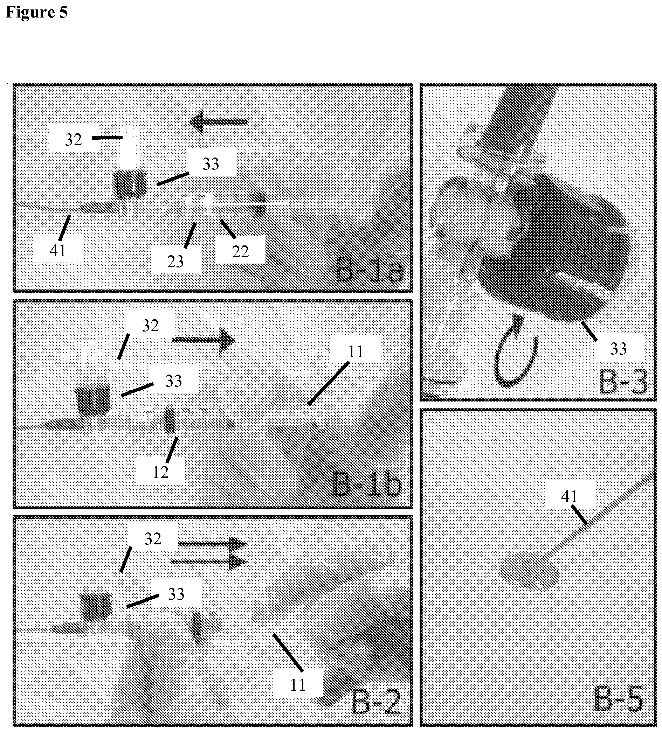

A hydrogel can be applied to dura mater (dura) with an applicator of Figure 1 as outlined below. With the vial (32) pointed up, the syringe plunger (11) is depressed to express all the liquid into the vial, the plunger is released and the device is shaken (Figure 5, B-1a). The plunger is allowed to recoil for 2-3 seconds while shaking (Figure 5, B-1b). This is repeated two more times to allow the powder to be dissolved and mixed. On the last (third) mixing cycle the plunger is pulled back to draw as much of the solution into the syringe as possible (Figure 5, B-2). The vial housing portion of the adapter (33) is unscrewed and discarded (Figure 5, B-3). This opens the fluid path to allow the solution to be dispensed through the cannula (41). Three full drops are expressed onto gauze by gently depressing the plunger. The cannula is now primed with solution and will allow for fine control of delivery. The applicator tip is oriented above the dura and the solution is expressed onto the surgical site (Figure 5, B-5). The surgical sealant (hydrogel) is applied until a thin coating (approximately 1 - 2 mm) is formed. To stop delivery, pressure on the plunger is released. It may be necessary to actively pull the plunger if too much pressure was initially applied. The solution polymerizes and cures. Excess hydrogel beyond the edges of the dural margin may be removed with a Penfield probe, scissors, or mechanical disruption.

### Example 5

Figure 6 shows an applicator that comprises a plunger (61) attached to a piston (62) which can be advanced through a syringe barrel. The syringe barrel is divided into an upper compartment (63) and a lower compartment (66) by a separator (64), which as shown is a hydrophobic barrier. Liquid can be placed in the upper compartment and can be pushed into the lower compartment when the piston advances towards the hydrophobic barrier. The lower compartment contains a dissolution aid (65) upon which a component of a formulation can be absorbed. The applicator further comprises a tip (67) which can be a blunt cannula. Also shown in Figure 6 is a vial (69), capped with a septum (68). The septum utilizes a cruciate slit which allows the blunt cannula to insert though the septum without undue force. Figure 6b shows the insertion of the tip into the vial through the septum.

### Example 6

One method of use for the applicator shown in Figure 6, described above in Example 5, is outlined below.

A user could begin by removing the cap on the syringe and replacing it with a blunt cannula (or other appropriate tip). The user would then insert the cannula through the slit in the vial, expel the liquid into the vial and shake to mix. After drawing the resulting mixed formulation back into the syringe, with the blunt cannula pointing up, the user could expel the air and the first portions of the mixed formulation to reduce the amount of head space in the syringe and to prime the cannula. The user could then apply the mixed formulation to a surface by manually depressing the piston of the syringe.

### Example 7

### Use of a prototype applicator kit (See Figures 15-19)

A prototype applicator kit was fabricated, including: instructions for use (IFU); the prototype applicator as shown in Figure 15, which includes two buffer-filled syringes, two piercing vial adapters with valves, a nozzle spray tip (dispensing tip), a battery powered micro air pump, and two AAA batteries; a first vial containing activated polyethylene glycol (PEG); and a second vial containing polyethyleneimine (PEI). The applicator may be supplied in a polymer tray for convenience and protection. As described below, the prototype applicator was assembled from the kit and then tested.

The vials containing activated PEG and PEI were placed onto their respective vial adapters, and the plungers of the two syringes were then indexed forward, pushing the two reconstitution buffers into the vials. The assembly was shaken until the two active ingredients were fully reconstituted (elapsed time of approximately 1 minute). The plungers of the two syringes were pulled back in order to recapture the reconstituted mixtures in the two syringes, and the two vial adapter hubs and vials were removed from the valves and set aside. The micro air pump was energized and the plungers of the double syringe were manually indexed forward using thumb pressure applied by the operator. This caused the liquids to move forward into the nozzle (dispensing tip) assembly, where the two liquids mixed, and then atomized by the air pressure and expelled out of the applicator onto a surface. The application of the gel was started and stopped several times without any detectable clogging of the nozzle (dispensing tip).

Several gel specimens were produced according to the protocol above, using either slow (gradual) introduction of the liquid into the pressurized air stream or fast (rapid) introduction of the liquid into the pressurized air stream. Under both circumstances the resulting gels solidified in approximately 1 second. The gel samples were then placed into balanced saline solution at a pH of 7.4 and incubated for 24 hours at 37° C. The incubated gels exhibited a percent weight gain ("swelling") of between 28.5 and 38.9%.

### Example 8

### Exemplary "Instructions for Use "for inclusion in prototype applicator kit (See Example 7; and Figures 15-19)

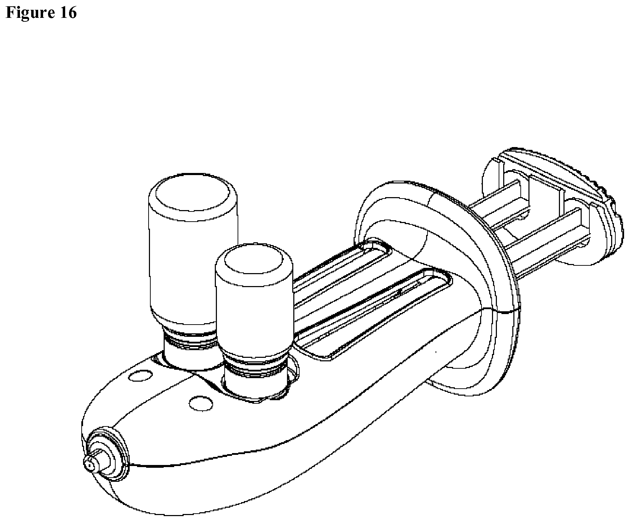

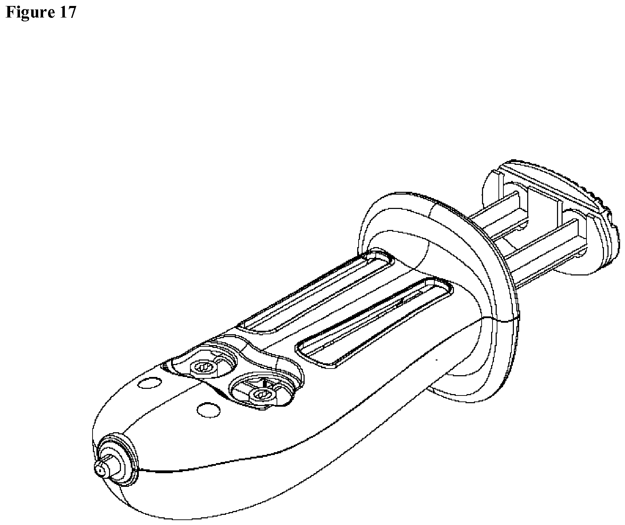

At time of use, the end user or an assistant shall perform the following procedure to assemble and use the applicator:
1. Remove the applicator and the two active-ingredient-containing glass vials from their respective sterile barrier packages.
2. Remove the two caps located on the spikes of the vial adapters.
3. Place the activated-PEG-containing vial onto the PEG-side vial adapter spike, and press until the spike completely pierces and captures the vial.
4. Place the PEI or PEI solution-containing vial onto the PEI-side vial adapter spike, and press until the spike completely pierces and captures the vial. The device should now look like Figure 16.
5. Manually index forward the double plunger so that the two reconstitution liquids are conveyed into the two vials.
6. Shake the applicator to ensure good reconstitution of the active ingredients; or draw the double plunger backwards and push the double plunger forward again several times to ensure good reconstitution of the active ingredients.
7. Once the active ingredients are reconstituted well, pull backwards on the double plunger to withdraw the solutions from their respective vials. Remove the vials and vial adapters from the valve assemblies by twisting the vial adapter hubs. Discard the two vial/vial adapter hub assemblies. Figure 17 shows the device at this stage.
8. Set aside the prepared device in the sterile field until ready for use. The device is stable in this configuration for at least 2 hours.
9. To apply sealant first turn on the micro air pump by pushing the electrical push button switch located on the underside of the device. Then, grasp the device as one would grasp a conventional syringe (i.e., with the body flanges placed between index and second fingers and thumb resting on the double plunger).
10. Direct the distal end of the device towards the desired surface of the patient, and begin application of the sealant by pushing the double plunger forward with thumb pressure. Application of the sealant may be temporarily interrupted or ceased by stopping the advance of the double plunger by removing thumb pressure.
11. The user may start and stop the device as many times as necessary to complete an application of the sealant onto the desired surface of the patient. In most cases, the user should apply a layer of sealant with thickness of 1-2 mm to provide a durable seal.
12. After application of the sealant has been completed, the batteries should be removed from the device for disposal in an appropriate waste stream. The rest of the device may be disposed in a standard hospital-waste stream.

## Claims

1. An applicator, comprising:
a first syringe barrel comprising a first sidewall; a first piston (12); a first proximal end; and a first distal end; wherein the first piston seals the first proximal end of the first syringe barrel and is slideable within the first syringe barrel; and the first distal end is configured to attach to a first adapter (31);
a second syringe barrel comprising a second sidewall; a second piston; a second proximal end; and a second distal end; wherein the second piston seals the second proximal end of the second syringe barrel and is slideable within the second syringe barrel; and the second distal end is configured to attach to a second adapter;
a first adapter comprising a first removable vial housing portion comprising a first piercing structure; a first syringe housing portion attached to the first distal end of the first syringe barrel; a first fitting configured to attach to a tip (41); a first valve; a first fluid pathway connecting the first syringe barrel to the first valve; a second fluid pathway connecting the first vial housing portion to the first valve; and a third fluid pathway connecting the first fitting to the first valve;
a second adapter comprising a second removable vial housing portion (33) comprising a second piercing structure; a second syringe housing portion attached to the second distal end of the second syringe barrel; a second fitting configured to attach to a tip; a second valve; a fourth fluid pathway connecting the second syringe barrel to the second valve; a fifth fluid pathway connecting the second vial housing portion to the second valve; and a sixth fluid pathway connecting the second fitting to the second valve;
a tip comprising a first inlet, a second inlet and an outlet, wherein the first inlet is connected to the first fitting, and the second inlet is connected to the second fitting;
a first component in the first syringe barrel;
a second component in the second syringe barrel; and
**characterized in that** the applicator further comprises a gas pump, wherein the gas pump is a built-in component of the applicator;
wherein the gas pump is adapted to assist with fluid movement within the applicator or from the tip or both.

2. The applicator of claim 1, wherein the gas pump is adapted to assist with fluid movement from the tip (41).

3. The applicator of claim 2, wherein the tip is a gas-assisted spray tip.

4. The applicator of any one of the preceding claims, wherein the gas pump is an air pump.

5. The applicator of any one of the preceding claims, wherein the gas pump is battery powered.

6. The applicator of claim 5, wherein the batteries are built-in.

7. The applicator of claim 5, wherein the batteries are removable.

8. A kit comprising the applicator of any one of the preceding claims.

9. The kit of claim 8, further comprising:
a first vial (32) containing a third component; and
a second vial containing a fourth component.

## Patentansprüche

1. Applikator, umfassend:
einen ersten Spritzenkörper, umfassend eine erste Seitenwand; einen ersten Kolben (12); ein erstes proximales Ende; und ein erstes distales Ende; wobei der erste Kolben das erste proximale Ende des ersten Spritzenkörpers abdichtet und im ersten Spritzenkörper gleiten kann; und das erste distale Ende konfiguriert ist, um an einem ersten Adapter (31) angebracht zu werden;
einen zweiten Spritzenkörper, umfassend eine zweite Seitenwand; einen zweiten Kolben; ein zweites proximales Ende; und ein zweites distales Ende; wobei der zweite Kolben das zweite proximale Ende des zweiten Spritzenkörpers abdichtet und im zweiten Spritzenkörper gleiten kann; und das zweite distale Ende konfiguriert ist, um an einem zweiten Adapter angebracht zu werden;
einen ersten Adapter, umfassend einen ersten entfernbaren Ampullengehäuseabschnitt, umfassend eine erste Durchstechstruktur; einen am ersten distalen Ende des ersten Spritzenkörpers angebrachten ersten Spritzenkörpergehäuseabschnitt; ein erstes Anschlussstück, das konfiguriert ist, um an eine Spitze (41) angebracht zu werden; ein erstes Ventil; einen ersten Fluiddurchlass, der den ersten Spritzenkörper mit dem ersten Ventil verbindet; einen zweiten Fluiddurchlass, der den ersten Ampullengehäuseabschnitt mit dem ersten Ventil verbindet; und einen dritten Fluiddurchlass, der das erste Anschlussstück mit dem ersten Ventil verbindet;
einen zweiten Adapter, umfassend einen zweiten entfernbaren Ampullengehäuseabschnitt (33), umfassend
eine zweite Durchstechstruktur; einen am zweiten distalen Ende des zweiten Spritzenkörpers angebrachten zweiten Spritzenkörpergehäuseabschnitt; ein zweites Anschlussstück, das konfiguriert ist, um an eine Spitze angebracht zu werden; ein zweites Ventil; einen vierten Fluiddurchlass, der den zweiten Spritzenkörper mit dem zweiten Ventil verbindet; einen fünften Fluiddurchlass, der den zweiten Ampullengehäuseabschnitt mit dem zweiten Ventil verbindet;
und einen sechsten Fluiddurchlass, der das zweite Anschlussstück mit dem zweiten Ventil verbindet;
eine Spitze, die einen ersten Einlass, einen zweiten Einlass und einen Auslass verbindet, wobei der erste Einlass mit dem ersten Anschlussstück verbunden ist und der zweite Einlass mit dem zweiten Anschlussstück verbunden ist;
eine erste Komponente im ersten Spritzenkörper;
eine zweite Komponente im zweiten Spritzenkörper; und **dadurch gekennzeichnet, dass** der Applikator ferner eine Gaspumpe umfasst, wobei die Gaspumpe eine eingebaute Komponente des Applikators ist;
wobei die Gaspumpe angepasst ist, die Fluidbewegung im Applikator oder von der Spitze oder beides zu unterstützen.

2. Applikator nach Anspruch 1, wobei die Gaspumpe angepasst ist, um die Fluidbewegung von der Spitze (41) zu unterstützen.

3. Applikator nach Anspruch 2, wobei die Spitze eine gasunterstützte Sprühspitze ist.

4. Applikator nach einem der vorstehenden Ansprüche, wobei die Gaspumpe eine Luftpumpe ist.

5. Applikator nach einem der vorstehenden Ansprüche, wobei die Gaspumpe batteriebetrieben ist.

6. Applikator nach Anspruch 5, wobei die Batterien eingebaut sind.

7. Applikator nach Anspruch 5, wobei die Batterien entfernbar sind.

8. Set, umfassend den Applikator nach einem der vorstehenden Ansprüche.

9. Set nach Anspruch 8, ferner umfassend:
eine erste Ampulle (32), enthaltend eine dritte Komponente; und eine zweite Ampulle, enthaltend eine vierte Komponente.

## Revendications

1. Applicateur, comprenant :
un premier cylindre de seringue comprenant une première paroi latérale ; un premier piston (12) ; une première extrémité proximale ; et une première extrémité distale ; ledit premier piston scellant la première extrémité proximale du premier cylindre de seringue et pouvant coulisser à l'intérieur du premier cylindre de seringue ; et la première extrémité distale étant conçue pour se fixer à un premier adaptateur (31) ;
un second cylindre de seringue comprenant une seconde paroi latérale ; un second piston ; une seconde extrémité proximale ; et une seconde extrémité distale ; ledit second piston scellant la seconde extrémité proximale du second cylindre de seringue et pouvant coulisser à l'intérieur du second cylindre de seringue ; et la seconde extrémité distale étant conçue pour se fixer à un second adaptateur ;
un premier adaptateur comprenant une première partie de logement de flacon amovible comprenant une première structure de perforation ; une première partie de logement de seringue fixée à la première extrémité distale du premier cylindre de seringue ; un premier embout conçu pour se fixer à une pointe (41) ; une première valve ; une première voie de passage de fluide reliant le premier cylindre de seringue à la première valve ; une deuxième voie de passage de fluide reliant la première partie de logement de flacon à la première valve ; et une troisième voie de passage de fluide reliant le premier embout à la première valve ;
un second adaptateur comprenant une seconde partie de logement de flacon amovible (33) comprenant une seconde structure de perforation ; une seconde partie de logement de seringue fixée à la seconde extrémité distale du second cylindre de seringue ; un second embout conçu pour se fixer à une pointe ; une seconde valve ; une quatrième voie de passage de fluide reliant le second cylindre de seringue à la seconde valve ; une cinquième voie de passage de fluide reliant la seconde partie de logement de flacon à la seconde valve ; et une sixième voie de passage de fluide reliant le second embout à la seconde valve ;
une pointe comportant une première entrée, une seconde entrée et une sortie, ladite première entrée étant reliée au premier embout, et ladite seconde entrée étant reliée au second embout ;
un premier composant dans le premier cylindre de seringue ;
un second composant dans le second cylindre de seringue ; et
**caractérisé en ce que** l'applicateur comprend en outre une pompe à gaz, ladite pompe à gaz étant un composant intégré de l'applicateur ;
ladite pompe à gaz étant adaptée pour aider au déplacement de fluide au sein de l'applicateur ou depuis la pointe ou les deux.

2. Applicateur selon la revendication 1, ladite pompe à gaz étant adaptée pour aider au déplacement de fluide depuis la pointe (41).

3. Applicateur selon la revendication 2, ladite pointe étant une pointe de pulvérisation assistée par gaz.

4. Applicateur selon l'une quelconque des revendications précédentes, ladite pompe à gaz étant une pompe à air.

5. Applicateur selon l'une quelconque des revendications précédentes, ladite pompe à gaz étant alimentée par batterie(s).

6. Applicateur selon la revendication 5, lesdites batteries étant intégrées.

7. Applicateur selon la revendication 5, lesdites batteries étant amovibles.

8. Kit comprenant l'applicateur selon l'une quelconque des revendications précédentes.

9. Kit selon la revendication 8, comprenant en outre :
un premier flacon (32) contenant un troisième composant ; et
un second flacon contenant un quatrième composant.
